(19) Europäisches Patentamt
European Patent Office
Office européen des brevets

(11) **EP 4 223 778 A1**

(12) **EUROPEAN PATENT APPLICATION**
published in accordance with Art. 153(4) EPC

(43) Date of publication:
**09.08.2023 Bulletin 2023/32**

(21) Application number: **21874470.4**

(22) Date of filing: **28.09.2021**

(51) International Patent Classification (IPC):
*C07K 16/28* (2006.01)    *C07K 16/46* (2006.01)
*C12N 15/13* (2006.01)    *C12N 15/63* (2006.01)
*A61K 39/395* (2006.01)    *A61P 35/00* (2006.01)

(52) Cooperative Patent Classification (CPC):
**A61K 39/395; A61P 35/00; C07K 16/00;
C07K 16/28; C07K 16/46; C12N 15/63**

(86) International application number:
**PCT/CN2021/121286**

(87) International publication number:
**WO 2022/068810 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2020 CN 202011054428**

(71) Applicant: **Innovent Biologics (Suzhou) Co., Ltd.
Suzhou, Jiangsu 215123 (CN)**

(72) Inventors:
• **ZHOU, Shuaixiang
Suzhou, Jiangsu 215123 (CN)**
• **GUAN, Zhe
Suzhou, Jiangsu 215123 (CN)**
• **WU, Weiwei
Suzhou, Jiangsu 215123 (CN)**
• **GAO, Yarong
Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **ANTI-CLAUDIN18.2 AND CD3 BISPECIFIC ANTIBODY AND USE THEREOF**

(57) The invention relates to novel bispecific antibodies specifically binding to Claudin 18.2 and CD3 and antibody fragments and compositions containing the antibodies or the antibody fragments. In addition, the invention relates to nucleic acids encoding the antibodies or antibody fragments thereof, host cells comprising the nucleic acids, and related uses. Furthermore, the present invention relates to the therapeutic and diagnostic uses of these antibodies and antibody fragments.

EP 4 223 778 A1

## Description

The invention relates to bispecific antibodies specifically binding to Claudin 18.2 and CD3 and compositions containing the said antibodies. In addition, the invention relates to nucleic acids encoding the said antibodies, host cells comprising the nucleic acids, and related uses. The invention further relates to the therapeutic and diagnostic uses of these antibodies and antibody fragments.

## Background of the Invention

Claudins is a protein family, which is an important component constituting the tight connection of cells. They establish an intercellular barrier to control the flow of molecules between cells, the barrier can control the flow of molecules between cells. Claudins family proteins have four transmembrane domains, and their N and C ends are included in the cytoplasm. Different Claudins proteins are expressed in different tissues, and their functional changes are related to the formation of cancer in various tissues. For example, Claudin-1 is expressed in colon cancer and has prognostic value, Claudin-18 is highly expressed in gastric cancer and pancreatic cancer, and Claudin-10 is highly expressed in hepatocellular carcinoma. As a cell membrane surface protein, Claudins is a useful target for various therapeutic strategies.

Claudin-18 allotype 2 (Claudin 18.2 or CLDN18.2) is a highly selective cell lineage marker. Its expression in normal tissues is strictly limited to epithelial cells differentiated from gastric mucosa, but not in gastric stem cells. CLDN18.2 is expressed in a considerable part of primary gastric cancer, and retains its expression level in gastric metastatic cancer tissues. In addition to gastric cancer, CLDN18.2 expression is also found in pancreatic cancer, which is an ideal target molecule for treating these cancers (Singh, P., Toom, S. & Huang, Y. Anti-CLDN18.2 antibody as new targeted therapy for advanced gastric cancer. J Hematol Oncol 10, 105 (2017). https://doi.org/10.1186/s13045-017-0473-4).

With regard to gastric cancer, in 2014, there were about 410000 new cases and 290000 deaths of gastric cancer nationwide, accounting for almost half of the global number of cases and deaths, and still showing an increasing trend. However, there are a large number of unmet clinical tumor treatment needs, so it is necessary to develop drugs targeting Claudin 18.2.

CD3 is a homodimer or heterodimer antigen expressed on T cells that combines with T cell receptor complex (TCR) and is required for T cell activation. Functional CD3 is formed by dimerization and association of two of the following four different chains: $\varepsilon$, $\zeta$, $\delta$ and $\gamma$. CD3 dimer arrangement comprises $\gamma/\varepsilon$, $\delta/\varepsilon$ and $\zeta/\zeta$. It has been shown that antibodies targeting CD3 aggregate CD3 on T cells, thus causing T cell activation in a manner similar to the way that MHC molecules loaded with peptides participate in TCR. Therefore, anti-CD3 antibodies have been proposed for therapeutic purposes involving T cell activation. In addition, it has been proposed that bispecific antibodies that can bind CD3 and target tumor surface antigens can connect tumor cells and T cells, thus directly activating T cells, releasing granzyme, perforin and cytokines to kill tumor, thus achieving the therapeutic purpose of inhibiting tumor. CLDN18.2 is highly expressed in gastric cancer, pancreatic cancer, and gastroesophageal junction cancer, etc., so it can be used to develop bispecific antibodies that combine CD3 and Claudin18.2 to achieve therapeutic purposes.

## Summary of The Invention

In some aspects, the invention provides a bispecific antibody, wherein the said antibody comprises two binding domains, wherein the first binding domain specifically binds to CLDN18.2, and the second binding domain specifically binds to CD3 .

In the preferred embodiment, the first binding domain specifically binding to CLDN18.2 of the invention is fully human, and/or the second binding domain is humanized.

In some aspects, the bispecific antibody of the invention is a bispecific antibody with IgG-like structure.

In one aspect, the invention relates to the following embodiments:

1. A bispecific antibody, which comprises a first antigen-binding domain and a second antigen-binding domain, wherein the first antigen-binding domain specifically binds to CLDN18.2, and the second antigen-binding domain specifically binds to CD3, wherein the first antigen-binding domain comprises three complementary determining regions A1-HCDR1, A1-HCDR2 and A1-HCDR3 contained in A1-VH as shown in SEQ ID NO: 4, and three complementary determining regions A1-LCDR1, A1-LCDR2 and A1-LCDR3 contained in VL as shown in SEQ ID NO: 9; the second antigen-binding domain comprises three complementary determining regions A2-HCDR1, A2-HCDR2 and A2-HCDR3 contained in A2-VH as shown in SEQ ID NO: 30, 22 or 32, and three complementary determining regions A2-LCDR1, A2-LCDR2 and A2-LCDR3 contained in A2-VL as shown in SEQ ID NO: 27.

2. The bispecific antibody of embodiment 1, wherein

the first antigen-binding domain comprises A1-HCDR1, A1-HCDR2, A1-HCDR3 as shown in the following amino acid sequence: SEQ ID NO: 1, 2 and 3, respectively, and A1-LCDR1, A1-LCDR2 and A1-LCDR3 as shown in the following amino acid sequence: SEQ ID NO: 6, 7 and 8, respectively; and

the second antigen-binding domain comprises

(i) A2-HCDR1, A2-HCDR2, A2-HCDR3 as shown in the following amino acid sequence: SEQ ID NO: 19, 20 and 29, respectively, and LCDR1, LCDR2 and LCDR3 as shown in the following amino acid sequence: SEQ ID NO: 24, 25 and 26, respectively; or

(ii) A2-HCDR1, A2-HCDR2, A2-HCDR3 as shown in the following amino acid sequence: SEQ ID NO: 19, 20 and 21, respectively, and LCDR1, LCDR2 and LCDR3 as shown in the following amino acid sequence: SEQ ID NO: 24, 25 and 26, respectively; or

(iii) A2-HCDR1, A2-HCDR2, A2-HCDR3 as shown in the following amino acid sequence: SEQ ID NO: 19, 31 and 21, respectively, and LCDR1, LCDR2 and LCDR3 as shown in the following amino acid sequence: SEQ ID NO: 24, 25 and 26, respectively.

3. The antibody of embodiment 1 or 2, the first antigen-binding domain comprises a heavy chain variable region and/or a light chain variable region, wherein the heavy chain variable region

(i) comprises or consists of an amino acid sequence having at least 90%, 91%, 92%, 93%, 94%, 95%, 96%, 97%, 98% or 99% identity with the amino acid sequence selected from SEQ ID NO: 4; or

(ii) comprises or consists of an amino acid sequence selected from SEQ ID NO: 4; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 4, preferably, the said amino acid changes do not occur in the CDR region; and/or

the light chain variable region

(i) comprises or consists of an amino acid sequence having at least 90%, 91%, 92%, 93%, 94%, 95%, 96%, 97%, 98% or 99% identity with the amino acid sequence selected from SEQ ID NO: 9; or

(ii) comprises or consists of an amino acid sequence selected from SEQ ID NO: 9; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 9, preferably, the said amino acid changes do not occur in the CDR region.

4. The antibody of any of embodiments 1-3, wherein the second antigen-binding domain comprises a heavy chain variable region and/or a light chain variable region, wherein the heavy chain variable region

(i) comprises or consists of an amino acid sequence having at least 90%, 91%, 92%, 93%, 94%, 95%, 96%, 97%, 98% or 99% identity with the amino acid sequence selected from SEQ ID NO: 30, 22 or 32; or

(ii) comprises or consists of an amino acid sequence selected from SEQ ID NO: 30, 22 or 32; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 30, 22 or 32, preferably, the said amino acid changes do not occur in the CDR region; and/or

the light chain variable region

(i) comprises or consists of an amino acid sequence having at least 90%, 91%, 92%, 93%, 94%, 95%, 96%, 97%, 98% or 99% identity with the amino acid sequence selected from SEQ ID NO: 27; or

(ii) comprises or consists of an amino acid sequence selected from SEQ ID NO: 27; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 27, preferably, the said amino acid changes do not occur in the CDR region.

5. The antibody of any of embodiments 1-4, further comprises a heavy chain constant region and/or a light chain constant region.

6. The antibody of any of embodiments 1-5, which is a bispecific antibody with IgG-like structure.

7. The antibody of any of embodiments 1-6, wherein the heavy chain constant region of the antibody is from IgG1 or IgG2 or IgG3 or IgG4, preferably from IgG1.

8. The antibody of any of embodiments 1-6, which comprises two heavy chain constant regions, one heavy chain constant region A1-HC is connected with the heavy chain variable region A1-VH of the first antigen domain to form the part of the heavy chain binding to CLDN18.2, and the other heavy chain constant region A2-HC is connected with the heavy chain variable region A2-VH of the second antigen binding domain to form the part of the heavy chain binding to CD3, and comprises two light chain constant regions, one light chain constant region A1-LC is connected with the light chain variable region A1-VL of the first antigen domain to form the part of the light chain binding to CLDN18.2, and the other light chain constant region A2-LC is connected with the light chain variable region A2-VL of the second antigen binding domain to form the part of the light chain binding to CD3.

9. The antibody of embodiment 8, wherein A1-HC can be the same or different from A2-HC, and/or A1-LC can be the same or different from A2-LC.

10. The antibody of embodiment 8, wherein A1-VH and A1-VL of the part binding to CLDN18.2 are fully human, and A2-VH and A2-VL of the part binding to CD3 are humanized.

11. The antibody of embodiment 8, wherein the part of the heavy chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 37;

(ii) comprises or consists of an amino acid sequence selected from SEQ ID NO: 37; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 37; and/or

the part of the light chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 38;

(ii) comprises or consists of an amino acid sequence selected from SEQ ID NO: 38; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 38.

12. The antibody of embodiment 8 or 11, wherein

the part of the heavy chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 41, 39 or 42;

(ii) comprises or consists of an amino acid sequence selected from SEQ ID NO: 41, 39 or 42; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 41, 39 or 42; and/or

the part of the light chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 40; or

(ii) comprises or consists of an amino acid sequence selected from SEQ ID NO: 40; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 40.

13. An isolated nucleic acid that encodes the light chain variable region or heavy chain variable region, or light chain or heavy chain of the antibody of any of embodiments 1 to 12.

14. A vector comprising the nucleic acid of embodiment 13, preferably the said vector is an expression vector.

15. A host cell comprising the nucleic acid of embodiment 13 or the vector of embodiment 14, preferably, the said host cell is prokaryotic or eukaryotic cell, more preferably selected from yeast cells, mammalian cells (such as 293 cells or CHO cells, such as CHO-S cells or HEK293 cells) or other cells suitable for preparing antibodies or antigen-binding fragments thereof.

16. A method for preparing an antibody binding to CLDN18.2 or antigen-binding fragment thereof, the said method comprises culturing the host cell of embodiment 15 under the conditions suitable for expressing the nucleic acid encoding the antibody of any of embodiment 1 to 12, optionally separating the antibody or antigen-binding fragment thereof, and optionally the said method further comprises recovering the said antibody from the said host cell.

17. A pharmaceutical composition, which comprises the antibody binding to CLDN18.2 or antigen-binding fragment thereof of any of embodiments 1 to 12, and optionally one or more other therapeutic agents, such as chemotherapeutic agents, angiogenesis inhibitors, cytokines, cytotoxic agents, other antibodies, small molecular drugs or immunomodulators (such as immune checkpoint inhibitors or agonists), and optionally a pharmaceutically acceptable supplementary material.

18. A pharmaceutical combination, which comprises the antibody or antigen-binding fragment thereof of any of embodiments 1 to 12, and optionally one or more other therapeutic agents, such as chemotherapeutic agents, angiogenesis inhibitors, cytokines, cytotoxic agents, other antibodies, small molecular drugs or immunomodulators (such as immune checkpoint inhibitors or agonists).

19. A method for preventing or treating tumor in a subject, the said method comprises administering to the subject an effective amount of the antibody or antigen-binding fragment thereof of any of embodiments 1 to 12, or the drug composition of embodiment 17, or the pharmaceutical combination of embodiment 18.

20. The method of embodiment 19, wherein the said tumor is a cancer, preferably, the said cancer has an elevated level (such as nucleic acid or protein level) of CLDN18.2, for example, the said cancer is pancreatic cancer or gastric cancer or gastroesophageal junction cancer.

21. The method of any of embodiments 19-20, the said method further comprises administering one or more therapies to the patient, such as therapeutic modes and/or other therapeutic agents, preferably the therapeutic modes comprise radiotherapy or surgery, or therapeutic agents comprise chemotherapy agents, angiogenesis inhibitors, cytokines, cytotoxic agents, other antibodies, small molecule drugs or immune modulators (such as immune checkpoint inhibitors or agonists).

**Description of the Drawings**

Figure 1 shows that HB37A6 antibody specifically binds to CLDN18.2 on the cell surface.

Figure 2 shows that HB37A6 antibody does not bind to CLDN18.1 on the cell surface.

Figure 3 shows the binding of HB37A6 antibody with gastric cancer cell line NUGC-4, gastric cancer cell line KATO III-hCLDN18.2 and pancreatic cancer cell line DAN-G-hCLDN18.2.

Figure 4 shows the anti-tumor effect of HB37A6 antibody in the pancreatic cancer mouse model.

Figure 5 shows the anti-tumor effect of HB37A6 antibody in the gastric cancer mouse model.

Figure 6 shows the binding affinity of CD3 monoclonal antibodies Hzsp34.24, Hzsp34.87 and Hzsp34.97 at the cell level.

Figure 7 shows the T cell activation ability of CD3 antibodies Hzsp34.24, Hzsp34.87 and Hzsp34.97.

Figure 8 shows the structure diagram of the bispecific antibody used in the examples.

Figure 9 shows that the bispecific antibody of the invention specifically kills CLDN18.2-positive gastric cancer cell NUGC-4.

Figure 10 shows that the bispecific antibody of the invention specifically kills CLDN18.2-positive pancreatic cancer cell DAN-GCLDN18.2.

Figure 11 shows that the bispecific antibody has no non-specific killing effect on CLDN18.2 negative cells.

Figure 12 shows the cytokine release mediated by T cells on which bispecific antibody depends in NUGC-4.

Figure 13 shows the cytokine release mediated by T cells on which bispecific antibody depends in DAN-G-CLDN18.2.

Figure 14 shows T-cell activation mediated by bispecific antibody on which CLDN18.2 expression depends.

Figure 15 shows the efficacy results of bispecific antibody in the humanized model of NUGC-4 gastric cancer in vivo.

Figure 16 shows the efficacy results of the bispecific antibody in the humanized model of DAN-G-CLDN18.2 pancreatic cancer in vivo.

Figure 17 shows the PK of bispecific antibodies in mice.

**Detailed Description of The Invention**

**I. Definition**

Before the invention is described in detail below, it should be understood that the invention is not limited to the particular methodology, protocols, and reagents described herein, as these may vary. It should also be understood that the terminology used herein is for the purpose of describing particular embodiments only, and is not intended to limit the scope of the invention, which will be limited only by the appended claims. Unless otherwise defined, all technical and scientific terms used herein have the same meaning as commonly understood by those of ordinary skill in the art to which the invention belongs.

For the purpose of explaining this specification, the following definitions will be used, and wherever appropriate, terms used in the singular may also include the plural and vice versa. It is understood that the terminology used herein is for the purpose of describing particular embodiments only and is not intended to be limiting.

The term "about" used in combination with a numerical value is intended to encompass the numerical values in a range from a lower limit less than the specified numerical value by 5% to an upper limit greater than the specified numerical value by 5%.

The term "and/or" as used herein, means any of the options or two or more of the options.

The term "comprise" or "include" as used herein means including the elements, integers or steps described, but does not exclude any other elements, integers or steps. The term also covers the combination of the elements, integers or steps mentioned herein when the term "comprises" or "include" is used, unless otherwise specified. For example, it is also intended to cover the antibody variable region composed of the specific sequence when referring to the antibody variable region "comprises" a specific sequence.

The term "CLAUDIN" or "CLDN" as used herein is the most important skeleton protein that determines the tight junction structure between cells, which participates in the adhesion connection and plays an important role in the metastasis and invasion of tumor cells. Claudin protein is widely distributed in mammalian epithelial and endothelial cells, and its distribution is mainly on the side of epithelial cells and on the plasma membrane of basal cells. Different Claudin proteins have their own specific expression in different tissues, wherein Claudin 18 (CLDN18) gene is located at 3q22.3, with a molecular weight of 24kDa, contains 261 amino acid residues, and belongs to the Claudins superfamily, its protein structure comprises 2 extracellular rings and 4 transmembrane regions. The two subtypes of human CLDN18 or Claudin18 protein are Claudin18.1 or CLDN18.1 (UniProt ID: P56856-1), and Claudin18.2 or CLDN18.2 (UniProt ID: P56856-2). In the primary structure sequence of the two proteins, only the amino acid residues from the N-terminal signal peptide to some positions of the extracellular loop 1 (Loop 1) structure are different, especially on the extracellular loop 1, CLDN18.1 and CLDN18.2 are only 8 amino acids different. The sequence homology of the two subtypes of CLDN18 protein between species and generais also very high. The sequence of the extracellular loop 1 of CLDN18.2 is completely consistent in different species such as human, mouse and macaque, while the homology of CLDN18.2 protein between human and mouse is 84%, indicating that the sequence of CLDN18.2 protein is extremely conservative (O. Tureci. et al., Gene 481:83-92, 2011). CLDN18.2 or any of variants and allotypes thereof can be isolated from cells or tissues that naturally express them, or recombined using techniques well known in the art and/or those techniques described herein. In one embodiment, CLDN18.2 described herein is human CLDN18.2.

The terms "anti-CLDN 18.2 antibody", "anti-CLDN18.2", "CLDN18.2 antibody" or " antibody binding to CLDN18.2" as used herein refer to such an antibody that can bind (human) CLDN18.2 with sufficient affinity so that the antibody can be used as a therapeutic agent targeting (human) CLDN18.2. In one embodiment, the (human) CLDN18.2 antibody binds (human) CLDN18.2 with high affinity in vitro or in vivo. In one embodiment, the (human) CLDN18.2 antibody does not bind to CLDN18.1. In one embodiment, the (human) CLDN18.2 antibody binds to the cells expressing CLDN18.2, but not to the cells expressing CLDN18.1. In some embodiments, the binding is measured, for example, by radioimmunoassay (RIA), biomembrane thin-layer interferometry (BLI), MSD assay or surface plasmon resonance (SPR) or flow cytometry.

The term "CD3" as used herein refers to the antigen expressed on T cells as part of the multi-molecule T cell receptor (TCR), and it is composed of homodimer or heterodimer formed by two of the following four receptor chains: CD3-$\varepsilon$, CD3-$\delta$, CD3-$\zeta$ and CD3-$\gamma$. Human CD3-$\varepsilon$n(hCD3$\varepsilon$) comprises the amino acid sequence described in UniProtKB/Swiss-Prot: P07766.2. Human CD3-$\delta$(hCD3 $\delta$) comprises the amino acid sequence described in UniProtKB/Swiss-Prot: P04234.1. In some embodiments, the CD3 described in the invention refers to CD3 from human or cynomolgus monkeys.

The term "CD3-binding antibody" or "anti-CD3 antibody" as used herein includes the antibody specifically recognizing or binding to a single CD3 subunit (e.g. $\varepsilon$, $\delta$, $\gamma$ or $\zeta$) and the antigen-binding fragment thereof, and the antibody specifically recognizing and binding to the dimer complex of two CD3 subunits (for example, $\gamma/\varepsilon$, $\delta/\varepsilon$ and $\zeta/\zeta$ CD3 dimer) and the antigen-binding fragment thereof. The antibody and antigen-binding fragment of the invention can bind to soluble CD3, binding CD3 and/or CD3 expressed on the cell surface. Soluble CD3 comprises natural CD3 protein and recombinant CD3 protein variants, such as monomer and dimer CD3 structures that lack transmembrane regions or otherwise do not bind to cell membranes. The invention provides antibodies that bind human and cynomolgus monkey CD3 with low or undetectable binding affinity to activate human and cynomolgus monkey T cells. In some embodiments, the binding is measured, for example, by radioimmunoassay (RIA), biomembrane thin-layer interferometry (BLI), MSD assay or surface plasmon resonance (SPR) or flow cytometry.

The term "CD3 expressed on the cell surface" refers to one or more CD3 proteins, which are expressed on the cell surface in vivo or in vitro, so that at least part of CD3 proteins is exposed to the outside of the cell membrane and are easy to approach the antigen-binding part of the antibody. "CD3 expressed on the cell surface" comprise CD3 protein contained in the functional T cell receptor environment in the cell membrane. The term "CD3 expressed on the cell surface" comprises CD3 protein expressed as a part of homodimer or heterodimer on the cell surface (for example, $\delta/\varepsilon$, $\gamma/\varepsilon$ and $\zeta/\zeta$ CD3 dimer).

The effector cells include effector T cells (T lymphocytes), such as CD4+T cells, CD8+T cells, Th1, Th2 and regulatory T cells (Tregs). Effector cells further comprise natural killer cells, macrophages, granulocytes, plasma cells or B cells (lymphocytes).

The term "multi-specific antibody" refers to an antibody that is at least bispecific, that is, the antibody comprises at least the first binding domain and the second binding domain, wherein the first binding domain binds one target or antigen and the second binding domain binds another antigen or target. Therefore, the antibody according to the invention comprises specificity for at least two different antigens or targets. The antibody according to the invention also covers a multi-specific antibody including a plurality of binding domains/binding sites, such as a trispecific antibody, wherein the antibody comprises three binding domains.

The bispecific antibody versions include IgG-like and non-IgG-like antibodies (Fan et al. (2015) Journal of Hematology & Oncology 8: 130). The most common IgG-like antibody type comprises two Fab regions and one Fc region. The heavy chain and light chain of each Fab can come from separate monoclonal antibodies. Non-IgG-like bispecific antibodies lack Fc region and each antigen or target binding domain thereof can be Fab, or single chain variable fragment (scFv), or fusion protein that simulates the variable domain of two antibodies. The different binding domains are joined together by peptide connector, chemical coupling, non-covalent bond connection or other ways. These versions comprise bispecific T-cell adaptor (BiTE).

Any bispecific antibody version or technology can be used to prepare the bispecific antibody of the invention. For example, an antibody or fragment thereof with the first antigen-binding specificity can be functionally joined with one or more other molecular entities such as another antibody or antibody fragment with the second antigen-binding specificity (for example, through chemical coupling, genetic fusion, non-covalent association or other ways) to produce bispecific antibodies. Specific example bispecific versions that can be used in the context of the invention include, but are not limited to, the following: scFv-based or dual-antibody bispecific versions, IgG-scFv fusion, dual-variable domain (DVD)-Ig, Quadroma, knobs-into-holes, ordinary light chains (for example, ordinary light chains with knobs, etc.), CrossMab, CrossFab, (SEED)body, Duobody, IgG1/IgG2, double-functional Fab (DAF)-IgG and Mab2 bispecific versions.

The term "linker" as used herein refers to any molecule that enables directly joining the different parts of a bispecific antibody. Examples of linker that establish covalent join between different antibody parts include peptide linker and non-protein liner, including but not limited to polyethylene glycol (PEG), polypropylene glycol, polyethylene oxide or copolymers of polyethylene glycol and polypropylene glycol.

The term "peptide linker" according to the invention refers to the sequence of amino acids, wherein the sequence joins the amino acid sequence of the first part of the antibody to the second part of the antibody. For example, the peptide linker may join the first (variable and/or binding) domain of the antibody to the second variable and/or binding) domain. For example, the peptide linker can also join one part of the antibody to another part of the antibody, such as join the antigen-binding domain to the Fc domain or fragment thereof. Preferably, the peptide liner has such a length that it is sufficient to join two entities in such a way that they maintain their conformation relative to each other, so as not to hinder the desired activity.

The peptide linker may or may not mainly include the following amino acid residues: Gly, Ser, Ala or Thr. Useful linkers include glycine-serine polymers, including, for example, $(GS)_n$, $(GSGGS)_n$, $(GGGGS)_n$, $(GGGS)_n$, and $(GGGGS)_nG$, where n is an integer of at least 1 (and preferably 2, 3, 4, 5, 6, 7, 8, 9, and 10). Useful linkers further include glycine-alanine polymer, alanine-serine polymer and other flexible linkers.

The term "valence" according to the invention means that there is a specified number of binding sites in the antibody molecule. Therefore, the terms bivalent, trivalent and tetravalent respectively indicate that there are two, three or four binding sites in the antibody construct. The bispecific antibody according to the invention is at least bivalent and can be multivalent, such as bivalent, trivalent, tetravalent or hexavalent.

The term "binding domain" as used herein refers to any part of a bispecific antibody that binds to a specific target or antigen. Binding domains are antigen-binding sites. The binding domain may be, for example, an antibody or immunoglobulin itself or an antibody fragment. Such binding domain may have or not have a tertiary structure independent of the rest of BsAB, and can be used as a separate entity binding to or not binding to its target.

In some exemplary embodiments of the invention, each antigen-binding domain of the bispecific antibody includes the heavy chain variable region VH and the light chain variable region VL. In the bispecific antibodies including the first antigen-binding domain and the second antigen-binding domain, the VH, VL or CDR of the first antigen-binding domain can be represented by the prefix "A1", and the VH, VL or CDR of the second antigen-binding domain can be represented by the prefix "A2". For example, the heavy chain CDR (HCDR) of the first antigen-binding domain can be called A1-HCDR1, A1-HCDR2 and A1-HCDR3 herein; and the heavy chain CDR of the second antigen-binding domain can be called A2-HCDR1, A2-HCDR2 and A2-HCDR3 herein. Similarly, the heavy chain variable region VH of the first antigen-binding domain is called A1-VH herein, and the heavy chain variable region VH of the second antigen-binding domain is called A2-VH herein. The light chain CDR (HCDR) of the first antigen-binding domain can be called A1-LCDR1, A1-LCDR2 and A1-LCDR3 herein; and the light chain CDR of the second antigen-binding domain can be called A2-LCDR1, A2-LCDR2 and A2-LCDR3 herein. Similarly, the light chain variable region VL of the first antigen-binding domain is called A1-VL herein, and the light chain variable region VL of the second antigen-binding domain is called A2-VL herein.

The term "antibody fragment" comprises a portion of the complete antibody. In the preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragment include but are not limited to Fv, Fab, Fab', Fab'-SH, F(ab')$_2$; dAb (domain antibody); a linear antibody; a single-chain variable fragment (e.g., scFv); a single-domain antibody, e.g., VHH; a bivalent antibody or a fragment thereof; a Camelidae antibody.

The term "antigen" refers to the molecule that triggers the immune response. Such immune response may involve antibody production or activation of specific immune cells, or both. Technicians will understand that any macromolecule, including basically all proteins or peptides, can be used as an antigen. In addition, antigens can be derived from recombinant or genomic DNA. The term "epitope" as used herein refers to the part of an antigen (e.g. CLDN18.2) that specifically interacts with the antibody molecule.

"Antibody that binds to the same or overlapping epitope" as a reference antibody refers to an antibody that blocks 50%, 60%, 70%, 80%, 90%, or 95% or more of the binding of the reference antibody to its antigen in a competition assay, or conversely, the reference antibody blocks 50%, 60%, 70%, 80%, 90%, or 95% or more of the binding of the antibody to its antigen in a competition assay.

An antibody that competes with a reference antibody to bind to its antigen refers to an antibody that blocks 50%, 60%, 70%, 80%, 90%, or 95% or more of the binding of the reference antibody to its antigen in a competition assay. Conversely, the reference antibody blocks 50%, 60%, 70%, 80%, 90%, or 95% or more of the binding of the antibody to its antigen in a competition assay. Numerous types of competitive binding assays can be used to determine whether an antibody competes with another, such as direct or indirect solid-phase radioimmunoassay (RIA), direct or indirect solid-phase enzyme immunoassay (EIA), and sandwich competition assay.

An antibody that inhibits (e.g., competitively inhibits) the binding of a reference antibody to its antigen refers to an antibody that inhibits 50%, 60%, 70%, 80%, 90%, or 95% or more of the binding of the reference antibody to its antigen. Conversely, the reference antibody inhibits 50%, 60%, 70%, 80%, 90%, or 95% or more of the binding of the antibody to its antigen. The binding of an antibody to its antigen can be measured by affinity (e.g., equilibrium dissociation constant). Methods for determining affinity are known in the art.

An antibody that shows the same or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any methods known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen contact residues ("antigen contact point"). CDRs are primarily responsible for binding to epitopes. The CDRs of the heavy and light chains are generally referred to as CDR1, CDR2, and CDR3, and are numbered sequentially from N-terminus. The CDRs located in the variable domain of the antibody heavy chains are referred to as HCDR1, HCDR2, and HCDR3, while the CDRs located in the variable domain of the antibody light chains are referred to as LCDR1, LCDR2, and LCDR3. In a given amino acid sequence of a light chain variable region or a heavy chain variable region, the exact amino acid sequence boundaries of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |

(continued)

| CDR | Kabat scheme | AbM scheme | Chothia scheme | Contact scheme |
|---|---|---|---|---|
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having the same Kabat numbering positions as a reference CDR sequence (e.g., any of the exemplary CDRs of the invention).

The term "CDR" or "CDR sequence" encompasses CDR sequences determined by any of the manners described above in the invention, unless otherwise stated.

Unless otherwise stated, in the invention, when referring to the positions of residues in an antibody variable region (including residues in a heavy chain variable region and residues in a light chain variable region), it refers to the numbering positions according to the Kabat numbering system (Kabat et al., Sequences of Proteins of Immunological Interest, 5th Ed. Public Health Service, National Institutes of Health, Bethesda, Md. (1991)).

In one embodiment, the heavy chain variable region CDR of the antibody of the invention is determined according to the following rules:

VH CDR1 is determined according to AbM rules; and VH CDR2 and 3 are determined according to Kabat rules.

In one embodiment, the light chain variable region CDR of the antibody of the invention is determined according to the Kabat rule.

In one embodiment, the heavy chain variable region CDR of the antibody of the invention is determined according to the following rules: VH CDR1 is determined according to the AbM rule; and VH CDR2 and 3 are determined according to Kabat rules; and the CDR of light chain variable area is determined according to Kabat rule.

It should be noted that boundaries of CDRs of variable regions of an antibody obtained by different assignment systems may differ. That is, CDR sequences of variable regions of an antibody defined by different assignment systems differ. Therefore, when it comes to defining an antibody with specific CDR sequences defined in the invention, the scope of the antibody also encompasses such antibody whose variable region sequences comprise the specific CDR sequences, but having claimed CDR boundaries different from the specific CDR boundaries defined by the invention as a different protocol (e.g., different assignment system rules or their combinations) is applied.

Antibodies with different specificities (i.e., different binding sites for different antigens) have different CDRs (under the same assignment system). However, although CDRs differ from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues of the rest CDR sequences can be determined by antibody structure and protein folding. Therefore, any variants of the CDRs given herein will also be considered in the invention. For example, in one CDR variant, the amino acid residues in the minimal binding unit may remain unchanged, while other CDR residues defined by Kabat or Chothia may be substituted by conservative amino acid residues.

The term "Fc region" is used herein to define the constant regions of CH2 and CH3 of the immunoglobulin heavy chain and the term includes the natural sequence Fc region and the variant Fc region. The natural Fc region can bind to different Fc receptors on the surface of immune cells, which can cause CDC\ADCC\ADCP effector function. Such effector functions generally require the combination of Fc region and binding domain (such as antibody variable domain). In some embodiments, the Fc region is mutated to enhance its CDC\ADCC\ADCP effector function. In some embodiments, the Fc region is mutated to weaken or delete its CDC\ADCC\ADCP effector function.

"Antibody in the form of IgG " refers to the IgG form that the heavy chain constant region of the antibody belonging to. Heavy chain constant regions of all antibodies of the same type are identical, and heavy chain constant regions of antibodies of different types are different. For example, an antibody in the form of IgG4 refers to the Ig domain of its heavy chain constant region from IgG4, or the antibody in the form of IgG1 refers to its heavy chain constant region from IgG1.

"Humanized" antibody refers to an antibody comprising amino acid residues from non-human CDR and human FR. In some embodiments, humanized antibodies will comprise basically all of at least one, usually two variable domains, where all or substantially all of the CDRs (for example, CDR) correspond to those of non-human antibodies, and all or substantially all of the FRs correspond to those of human antibodies. The humanized antibody can optionally comprise at least a portion of the antibody constant region derived from the human antibody. The "humanized form" of antibody (such as non-human antibody) refers to the antibody that has been humanized.

"Human antibody" or "whole human antibody" or "fully human antibody" can be used interchangeably and it refers to the antibody with such amino acid sequence, the said amino acid sequence corresponds to the amino acid sequence of the following antibody. The said antibody is generated by human or human cells or comes from non-human sources, which uses human antibody library or other human antibody coding sequence. Such definition of human antibody clearly excludes humanized antibodies containing non-human antigen-binding residues.

The term "binding" or "specific binding" as used herein means that binding interactions to antigen are selective and can be distinguished from unwanted or non-specific interactions. The ability of antigen binding sites to bind to specific antigens can be determined by enzyme-linked immunosorbent assay (ELISA) or conventional binding assay known in the art, such as radioimmunoassay (RIA), thin-layer biomembrane interference assay, MSD assay or surface plasmon resonance (SPR).

The term "therapeutic agent" as described herein comprises any substance effective in preventing or treating tumors (such as cancer), including a chemotherapeutic agent, a cytokine, an angiogenesis inhibitor, a cytotoxic agent, other antibodies, a small molecule drug or an immunomodulatory agent (such as an immunosuppressant).

The term "cytotoxic agent" used in the invention refers to a substance that inhibits or prevents the cell function and/or causes cell death or destruction.

"Chemotherapeutic agents" include chemical compounds useful in treatment of cancer or immune system disease.

The term "small molecule drugs" refers to organic compounds with low molecular weight that can regulate biological processes. "Small molecule" is defined as a molecule with molecular weight less than 10kD, generally less than 2kD and preferably less than 1kD. Small molecules include but are not limited to inorganic molecules, organic molecules, organic molecules containing inorganic components, molecules containing radioactive atoms, synthetic molecules, peptide mimics and antibody mimics. As a therapeutic agent, small molecules can penetrate cells more easily than large molecules, and are less susceptible to degradation and less prone to trigger immune response.

The term "immunomodulators" as used herein refer to natural or synthetic active agents or drugs that inhibit or regulate immune response. The immune response can be humoral or cellular. Immunomodulators include immunosuppressants. In some embodiments, the immune modulator of the invention includes an immune checkpoint inhibitor or an immune checkpoint agonist.

The term "effective amount" refers to the amount or dose of the antibody or fragment or composition or combination of the invention, which will produce the expected effect in patients needing such treatment or prevention after being administered to patients in a single or multiple dose.

"Therapeutically effective amount" refers to the amount that can effectively achieve the desired results at the required dose and for the required period of time. The therapeutically effective amount is also such an amount, where any toxic or harmful effect of antibody or antibody fragment or composition or combination is less than the therapeutic beneficial effect. "Therapeutically effective amount" preferably inhibits measurable parameters (such as tumor volume) by at least about 40%, or even more preferably by at least 50%, 55%, 60%, 65%, 70%, 75%, 80%, 85%, 90%, or even 100% compared to untreated objects.

"Preventively effective amount" refers to the amount that can effectively achieve the desired prevention results at the required dose and for the required period of time. Generally, since the preventive dose is used before or at an earlier stage of the disease in the objects, the preventively effective amount will be less than the therapeutically effective amount.

The terms "host cell", "host cell line" and "host cell culture" are used interchangeably and refer to the cells in which foreign nucleic acids are introduced, including the descendants of such cells. Host cells include "transformants" and "transformed cells", which include primary transformed cells and offspring derived from them, regardless of the number of passages. The nucleic acid content of the offspring may not be exactly the same as that of the parent cell, but may contain mutations. The mutant progeny with the same function or biological activity screened or selected from the initially transformed cells are included herein.

The term "label" as used herein refers to a compound or composition that is directly or indirectly conjugated or fused to a reagent (such as a polynucleotide probe or antibody) and facilitates the detection of the conjugated or fused reagent. The label itself can be detectable (for example, radioisotope label or fluorescent label) or can catalyze the chemical changes of detectable substrate compounds or compositions in the case of enzymatic labeling. The term is intended to cover the direct labeling of probes or antibodies by coupling (i.e., physically connecting) detectable substances to probes or antibodies and the indirect labeling of probes or antibodies by reacting with another directly labeled reagent.

"Individuals" or "subjects" include mammals. Mammals include, but are not limited to, domestic animals (such as cattle, sheep, cats, dogs and horses), primates (such as human and non-human primates, such as monkeys), rabbits, and rodents (such as mice and rats). In some embodiments, the individuals or subjects are human.

"Isolated" antibodies are antibodies that have been separated from their natural environment components. In some embodiments, the antibody is purified to more than 95% or 99% purity, such as by electrophoresis (for example, SDS-PAGE, isoelectric focusing (IEF), capillary electrophoresis) or chromatography (for example, ion exchange or reverse phase HPLC).

" Isolated nucleic acid encoding anti-CLDN18.2xCD3 bispecific antibody or fragments thereof" refers to one or more nucleic acid molecules, which encode the heavy chain or light chain of the antibody (or fragments thereof, such as the heavy chain variable region or light chain variable region), including such nucleic acid molecules in a single vector or separate vectors, and such nucleic acid molecules present in one or more positions in the host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in the first and second amino acid sequences or in one or both of nucleic acid sequences, or non-homologous sequences can be discarded for comparison purposes). In one preferred embodiment, for comparison purposes, the length of the aligned reference sequence is at least 30%, preferably at least 40%, more preferably at least 50%, 60%, and even more preferably at least 70%, 80%, 90%, 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, then the molecules are identical at this position.

A mathematical algorithm can be used to achieve the sequence comparison and calculation of percent identity between two sequences. In one preferred embodiment, the percent identity between two amino acid sequences is determined with the Needlema and Wunsch ((1970) J. Mol. Biol., 48:444-453) algorithm (available at http://www.gcg.com) which has been integrated into the GAP program of the GCG software package, using the Blossom 62 matrix or PAM250 matrix and gap weights of 16, 14, 12, 10, 8, 6, or 4 and length weights of 1, 2, 3, 4, 5, or 6. In yet another preferred embodiment, the percent identity between two nucleotide acid sequences is determined with the GAP program (available at http://www.gcg.com) of the GCG software package, using the NWSgapdna. CMP matrix and gap weights of 40, 50, 60, 70, or 80 and length weights of 1, 2, 3, 4, 5 or 6. A particularly preferred parameter set (and one that should be used unless otherwise stated) is a Blossom 62 scoring matrix with a gap penalty of 12, a gap extension penalty of 4, and a frameshift gap penalty of 5. The percent identity between two amino acid sequences or nucleotide sequences can also be determined with PAM120 weighted remainder table, gap length penalty of 12 and gap penalty of 4, using the E. Meyers and W. Miller algorithms which have been incorporated into the ALIGN program (version 2.0) ((1989) CABIOS, 4:11-17). Additionally or optionally, the nucleic acid sequences and protein sequences described herein can be further used as "query sequences" to perform searches against public databases to, e.g., identify other family member sequences or related sequences.

As used herein, the term "hybridization under stringent conditions, such as conditions of low stringency, medium stringency, high stringency, or extreme stringency" describes hybridization and washing conditions. Instructions for performing hybridization reactions can be found in Current Protocols in Molecular Biology, John Wiley & Sons, N.Y (1989), 6.3.1-6.3.6, which is incorporated by reference. Aqueous and non-aqueous methods are described in the references and either method can be used. The specific hybridization conditions mentioned herein are as followed: 1) low stringency hybridization conditions are in 6 X sodium chloride/sodium citrate (SSC) at about 45 °C, followed by two washes in 0.2 X SSC, 0.1% SDS at least at 50 °C (for low stringency conditions, the temperature of the washes can be increased to 55 °C); 2) medium stringency hybridization conditions are in 6 X SSC at about 45 °C, followed by one or more washes in 0.2 X SSC, 0.1% SDS at about 60 °C; 3) high stringency hybridization conditions are in 6 X SSC at about 45 °C, followed by one or more washes in 0.2 X SSC, 0.1% SDS at 65 °C; and preferably 4) extreme stringency hybridization conditions are in 0.5 M sodium phosphate, 7% SDS at 65 °C, followed by one or more washes in 0.2X SSC, 0.1% SDS at 65 °C. Extreme stringency condition (4) is a preferred condition and the one that should be used unless otherwise stated.

The term "anti-tumor effect" refers to biological effects that can be demonstrated by various means, including but not limited to, for example, reduction of tumor volume, tumor cell number tumor cell proliferation or tumor cell survival.

The terms "tumor" and "cancer" are used interchangeably herein, covering solid tumors and blood tumors.

The terms "cancer" and "cancerous" refer to or describe physiological diseases in mammals characterized by unregulated cell growth. In some embodiments, cancers suitable for treatment by the antibodies of the invention include gastric cancer, pancreatic cancer, or gastroesophageal junction cancer, including metastatic forms of those cancers.

The term "tumor" refers to the growth and proliferation of all neoplastic cells, whether malignant or benign, as well as all pre-cancerous and cancerous cells and tissues. The terms "cancer", "cancerous" and "tumor" are not mutually exclusive when mentioned herein.

The term "pharmaceutically acceptable supplementary material" refers to diluents, adjuvants (e.g., Freund's adjuvants (complete and incomplete)), excipients, carriers, or stabilizers, etc., which are co-administered with active substance.

The term "pharmaceutical composition" refers to such a composition that exists in a form which allows the biological activity of the active ingredient contained therein to be effective, and does not comprise additional ingredients having unacceptable toxicity to a subject to which the composition is administered.

The term "pharmaceutical combination" refers to non-fixed combination products or fixed combination products, including but not limited to drug kits and drug compositions. The term "unfixed combination" means that the active ingredients (for example, (i) the anti-CLDN18.2 x CD3 bispecific antibody or fragments thereof in the invention, and (ii) other therapeutic agents) are administered to patients simultaneously, without specific time limits or at the same or different time intervals, in sequence, in separate entities, where these two or more active agents are administered to provide effective levels of prevention or treatment in patients. In some embodiments, the anti-CLDN18.2 x CD3 bispecific antibody or fragments thereof and other therapeutic agents of the invention used in the pharmaceutical combination are administered at a level not exceeding the level when they are used alone. The term "fixed combination" means that two or more active agents are administered simultaneously to patients in the form of a single entity. It is preferred to select the dose and/or time interval of two or more active agents, so that the combined use of each component can produce greater effect than the single use of any one component in the treatment of disease or disorder. Each component can take its own form of preparation, which can be the same or different.

The term "combination therapy" refers to the application of two or more therapeutic agents or therapeutic modes (such as radiotherapy or surgery) to treat the diseases described herein. Such administration includes the co-administration of these therapeutic agents in a substantially simultaneous manner, such as in a single capsule with a fixed proportion of active ingredients. Alternatively, such application includes the joint application of each active ingredient in multiple or separate containers (such as tablets, capsules, powders and liquids). The powder and/or liquid can be reconstituted or diluted to the required dose before application. In addition, this application also includes the use of each type of therapeutic agent at approximately the same time or at different times in a sequential manner. In either case, the treatment plan will provide the beneficial effect of pharmaceutical combination in treating the disease or condition described herein.

As used herein, "treatment" (or "treat" or "treating") refers to slowing, interrupting, arresting, alleviating, stopping, reducing, or reversing the progression or severity of an existing symptom, disorder, condition, or disease.

As used herein, "prevention" (or "prevent" or "preventing") includes the inhibition of the onset or progression of a disease or disorder or a symptom of a particular disease or disorder. In some embodiments, subjects with family history of cancer are candidates for preventive regimens. Generally, in the context of cancer, the term "prevention" refers to the administration of a drug prior to the onset of signs or symptoms of a cancer, particularly in subjects at risk of cancer.

The term "vector" as used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

"Subject/patient/individual sample" refers to a collection of cells or fluids obtained from a patient or subject. The source of the tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; cells from a subject at any time during pregnancy or development. Tissue samples may comprise compounds which are naturally not mixed with tissues, such as preservatives, anticoagulants, buffers, fixatives, nutrients, antibiotics, and the like.

## II. Antibodies

In some embodiments, the anti-CLDN18.2 x CD3 bispecific antibody of the invention binds to CLDN18.2 (for example, human CLDN18.2) with high affinity. In some embodiments, the antibody of the invention specifically binds to CLDN18.2 (such as human CLDN18.2), but does not bind to CLDN18.1 (such as human CLDN18.1). In some embodiments, the binding affinity of the antibody or its antigen-binding fragment of the invention with human CLDN18.2 is higher than that of the known CLDN18.2 antibody, such as Zolbetuximab (Zmab) antibody. In some embodiments, the anti-CLDN18.2 x CD3 bispecific antibody of the invention binds to CD3 (such as human CD3 or cynomolgus monkey CD3) with the required affinity (such as low). In some embodiments, the antibody of the invention can bind human CD3 and cynomolgus monkey CD3 both. In some embodiments, the affinity of the antibody is determined by thin layer interferometry or surface plasmon resonance.

In some embodiments, the anti-CLDN18.2 x CD3 bispecific antibody of the invention can bind CLDN18.2 on the surface of tumor cells and CD3 on the surface of effector cells. In some embodiments, the binding is detected by flow cytometry.

In some embodiments, the antibody of the invention can specifically kill tumor cells, such as tumor cells expressing CLDN18.2. In some embodiments, the antibody of the invention can mediate cytokines, such as IL-2 and TNF α and/or release of IFN-γ. In some embodiments, the antibody of the invention can activate effector cells, such as T cells.

In some embodiments, the effector cells are T cells, such as T lymphocytes, such as CD4+T cells or CD8+T cells.

In some embodiments, the antibody or the antigen-binding fragment thereof of the invention can be used to treat cancer. In some embodiments, the antibody or antigen-binding fragment thereof of the invention can effectively inhibit tumor growth, and the tumor inhibition rate is greater than or equal to about 25%, 30%, 35%, 40%, 45%, 50%, 55%, 60%, 65%, 70%, 75%, 80%, 85%, 90%, 95%, or even 100%.

In some embodiments, the bispecific antibody of the invention comprises the first antigen-binding domain and the second antigen-binding domain, wherein the first antigen-binding domain specifically binds CLDN18.2 and the second antigen-binding domain specifically binds CD3.

In the preferred embodiment of the invention, the first antigen-binding domain comprises three complementary determining regions (A1-HCDR) from the heavy chain variable region, A1-HCDR1, A1-HCDR2 and A1-HCDR3.

In the preferred embodiment of the invention, the first antigen-binding domain comprises three complementary determining regions (A1-LCDR) from the light chain variable region, A1-LCDR1, A1-LCDR2 and A1-LCDR3.

In some embodiments, the first antigen-binding domain comprises three complementary determining regions (A1-HCDR) from the heavy chain variable region and three complementary determining regions (A1-LCDR) from the light chain variable region.

In some aspects, the first antigen-binding domain comprises the heavy chain variable region (A1-VH). In some aspects, the first antigen-binding domain comprises a light chain variable region (A1-VL). In some aspects, the first antigen-binding domain includes a heavy chain variable region and a light chain variable region. In some embodiments, the heavy chain variable region includes three complementary determining regions (A1-HCDR) from the heavy chain variable region, HCDR1, HCDR2 and HCDR3. In some embodiments, the light chain variable region includes three complementary determining regions (A1-LCDR) from the light chain variable region, LCDR1, LCDR2 and LCDR3.

In some embodiments, A1-VH

(i) comprises or consists of an amino acid sequence having at least 90%, 91%, 92%, 93%, 94%, 95%, 96%, 97%, 98% or 99% identity with the amino acid sequence selected from SEQ ID NO: 4; or

(ii) comprises or consists of an amino acid sequence selected from SEQ ID NO: 4; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 4, preferably, the said amino acid changes do not occur in the CDR region.

In some embodiments, A1-VL

(i) comprises or consists of an amino acid sequence having at least 90%, 91%, 92%, 93%, 94%, 95%, 96%, 97%, 98% or 99% identity with the amino acid sequence selected from SEQ ID NO: 9; or

(ii) comprises or consists of an amino acid sequence selected from SEQ ID NO: 9; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 9, preferably, the said amino acid changes do not occur in the CDR region.

In some embodiments, the three complementary decision regions (A1-HCDR) from A1-VH of the invention, A1-HCDR1, A1-HCDR2 and A1-HCDR3 are

(i) the three complementary determining regions A1-HCDR1, A1-HCDR2 and A1-HCDR3 contained in VH as shown in SEQ ID NO: 4, or

(ii) the three A1-HCDR regions comprise a sequence with at least one but no more than 5, 4, 3, 2 or 1 amino acid change (preferably amino acid substitution, preferably conservative substitution) compared to the sequence in (i).

In some embodiments, the three complementary decision regions (A1-LCDR) from A1-VL of the invention, A1-LCDR1, A1-LCDR2 and A1-LCDR3 are

(i) the three complementary determining regions A1-LCDR1, A1-LCDR2 and A1-LCDR3 contained in VL as shown in SEQ ID NO: 9, or

(ii) the three A1-LCDR regions comprise a sequence with at least one but no more than 5, 4, 3, 2 or 1 amino acid change (preferably amino acid substitution, preferably conservative substitution) compared to the sequence in (i).

In some embodiments, A1-HCDR1 comprises or consists of the amino acid sequence of SEQ ID NO: 1, or A1-HCDR1 comprises an amino acid sequence that has one, two or three changes (preferably amino acid substitution, preferably conservative substitution) compared to the amino acid sequence of SEQ ID NO: 1.

In some embodiments, A1-HCDR2 comprises or consists of the amino acid sequence of SEQ ID NO: 2, or A1-HCDR2 comprises an amino acid sequence that has one, two or three changes (preferably amino acid substitution, preferably conservative substitution) compared to the amino acid sequence of SEQ ID NO: 2.

In some embodiments, A1-HCDR3 comprises or consists of the amino acid sequence of SEQ ID NO: 3, or A1-HCDR3 comprises an amino acid sequence that has one, two or three changes (preferably amino acid substitution, preferably conservative substitution) compared to the amino acid sequence of SEQ ID NO: 3.

In some embodiments, A1-LCDR1 comprises or consists of the amino acid sequence of SEQ ID NO: 6, or A1-LCDR1 comprises an amino acid sequence that has one, two or three changes (preferably amino acid substitution, preferably conservative substitution) compared to the amino acid sequence of SEQ ID NO: 6.

In some embodiments, A1-LCDR2 comprises or consists of the amino acid sequence of SEQ ID NO: 7, or A1-LCDR2 comprises an amino acid sequence that has one, two or three changes (preferably amino acid substitution, preferably conservative substitution) compared to the amino acid sequence of SEQ ID NO: 7.

In some embodiments, A1-LCDR3 comprises or consists of the amino acid sequence of SEQ ID NO: 8, or A1-LCDR3 comprises an amino acid sequence that has one, two or three changes (preferably amino acid substitution, preferably conservative substitution) compared to the amino acid sequence of SEQ ID NO: 8.

In the preferred embodiment of the invention, the second antigen-binding domain comprises three complementary determining regions (A2-HCDR) from the heavy chain variable region, A2-HCDR1, A2-HCDR2 and A2-HCDR3.

In the preferred embodiment of the invention, the second antigen-binding domain comprises three complementary determining regions (A2-LCDR) from the heavy chain variable region, A2-LCDR1, A2-LCDR2 and A2-LCDR3.

In some embodiments, the second antigen-binding domain comprises three complementary determining regions (A2-HCDR) from the heavy chain variable region and three complementary determining regions (A2-LCDR) from the light chain variable region.

In some aspects, the second antigen-binding domain comprises the heavy chain variable region (A2-VH). In some aspects, the second antigen-binding domain comprises a light chain variable region (A2-VL). In some aspects, the second antigen-binding domain includes a heavy chain variable region and a light chain variable region. In some embodiments, the heavy chain variable region includes three complementary determining regions (A2-HCDR) from the heavy chain variable region, HCDR1, HCDR2 and HCDR3. In some embodiments, the light chain variable region includes three complementary determining regions (A2-LCDR) from the light chain variable region, LCDR1, LCDR2 and LCDR3.

In some embodiments, A2-VH

(i) comprises or consists of an amino acid sequence having at least 90%, 91%, 92%, 93%, 94%, 95%, 96%, 97%, 98% or 99% identity with the amino acid sequence selected from SEQ ID NO: 22, 30 or 32; or

(ii) comprises or consists of an amino acid sequence selected from SEQ ID NO: 22, 30 or 32; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 22, 30 or 32, preferably, the said amino acid changes do not occur in the CDR region.

In some embodiments, A2-VL

(i) comprises or consists of an amino acid sequence having at least 90%, 91%, 92%, 93%, 94%, 95%, 96%, 97%, 98% or 99% identity with the amino acid sequence selected from SEQ ID NO: 27; or

(ii) comprises or consists of an amino acid sequence selected from SEQ ID NO: 27; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 27, preferably, the said amino acid changes do not occur in the CDR region.

In some embodiments, the three complementary decision regions (A2-HCDR) from A2-VH of the invention, A2-HCDR1, A2-HCDR2 and A2-HCDR3 are

(i) the three complementary determining regions A2-HCDR1, A2-HCDR2 and A2-HCDR3 contained in VH as shown in SEQ ID NO: 20, 30 or 32, or

(ii) the three A2-HCDR regions comprise a sequence with at least one but no more than 5, 4, 3, 2 or 1 amino acid change (preferably amino acid substitution, preferably conservative substitution) compared to the sequence in (i).

In some embodiments, the three complementary decision regions (A2-LCDR) from A2-VL of the invention, A2-LCDR1, A2-LCDR2 and A2-LCDR3 are

(i) the three complementary determining regions A2-LCDR1, A2-LCDR2 and A2-LCDR3 contained in A2-VL as shown in SEQ ID NO: 27, or

(ii) the three A2-LCDR regions comprise a sequence with at least one but no more than 5, 4, 3, 2 or 1 amino acid change (preferably amino acid substitution, preferably conservative substitution) compared to the sequence in (i).

In some embodiments, A2-HCDR1 comprises or consists of the amino acid sequence of SEQ ID NO: 19, or A2-HCDR1 comprises an amino acid sequence that has one, two or three changes (preferably amino acid substitution, preferably conservative substitution) compared to the amino acid sequence of SEQ ID NO: 19.

In some embodiments, A2-HCDR2comprises or consists of the amino acid sequence of SEQ ID NO: 20 or 31, or A2-HCDR2 comprises an amino acid sequence that has one, two or three changes (preferably amino acid substitution, preferably conservative substitution) compared to the amino acid sequence of SEQ ID NO: 20 or 31.

In some embodiments, A2-HCDR3 comprises or consists of the amino acid sequence of SEQ ID NO: 21 or 29, or A2-HCDR3 comprises an amino acid sequence that has one, two or three changes (preferably amino acid substitution, preferably conservative substitution) compared to the amino acid sequence of SEQ ID NO: 21 or 29.

In some embodiments, A2-LCDR1 comprises or consists of the amino acid sequence of SEQ ID NO: 24, or A2-LCDR1 comprises an amino acid sequence that has one, two or three changes (preferably amino acid substitution, preferably conservative substitution) compared to the amino acid sequence of SEQ ID NO: 24.

In some embodiments, A2-LCDR2 comprises or consists of the amino acid sequence of SEQ ID NO: 25, or A2-LCDR2 comprises an amino acid sequence that has one, two or three changes (preferably amino acid substitution, preferably conservative substitution) compared to the amino acid sequence of SEQ ID NO: 25.

In some embodiments, A2-LCDR3 comprises or consists of the amino acid sequence of SEQ ID NO: 26, or A2-LCDR3 comprises an amino acid sequence that has one, two or three changes (preferably amino acid substitution, preferably conservative substitution) compared to the amino acid sequence of SEQ ID NO: 26.

In some embodiments, the bispecific antibody of the invention also comprises a heavy chain constant region. In some embodiments, the bispecific antibody of the invention also comprises a light chain constant region. In some embodiments, the bispecific antibody of the invention also comprises a heavy chain constant region and a light chain constant region. In some embodiments, the bispecific antibody of the invention comprises two heavy chain constant regions, one of which A1-HC is linked to the heavy chain variable region A1-VH of the first antigen domain to form the part of the heavy chain binding to the CLDN18.2, and the other A2-HC is linked to the heavy chain variable region A2-VH of the second antigenic binding domain to form the part of the heavy chain binding to the CD3. In some embodiments, the bispecific antibody of the invention comprises two light chain constant regions, one of which is linked to the light chain variable region A1-VL of the first antigen domain to form the part of the light chain binding to the CLDN18.2, and the other light chain constant region A2-LC is linked to the light chain variable region A2-VL of the second antigenic binding domain to form the part of the light chain binding to CD3. In one embodiment, the bispecific antibody of the invention comprises A1-HC, A1-LC, A2-HC and A2-LC. In some embodiments, A1-HC can be the same or different from A2-HC. In some embodiments, A1-LC can be the same or different from A2-LC. In some embodiments, A1-HC is different from A2-HC, and A1-LC is different from A2-LC.

In some embodiments, the heavy chain constant region HC of the invention is the heavy chain constant region of IgG1, IgG2, IgG3 or IgG4, and the preferred heavy chain constant region of IgG1, such as wild type IgG1 heavy chain constant region or mutant IgG1 heavy chain constant region (such as IgG1 LALA). In some embodiments, the antibody light chain constant region LC of the invention is lambda or Kappa light chain constant region.

In some embodiments, the heavy chain constant region HC of the invention

(i) comprises or consists of an amino acid sequence having at least 85%, 90%, 91%, 92%, 93%, 94%, 95%, 96%, 97%, 98% or 99% identity with the amino acid sequence selected from SEQ ID NO: 5 or 23; or

(ii) comprises or consists of an amino acid sequence selected from SEQ ID NO: 5 or 23; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 5 or 23.

In some embodiments, the light chain constant region HC of the invention

(i) comprises or consists of an amino acid sequence having at least 85%, 90%, 91%, 92%, 93%, 94%, 95%, 96%, 97%, 98% or 99% identity with the amino acid sequence selected from SEQ ID NO: 33 or 34; or

(ii) comprises or consists of an amino acid sequence selected from SEQ ID NO: 33 or 34; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 33 or 34.

In some embodiments, the light chain constant region LC of the invention

(i) comprises or consists of an amino acid sequence having at least 85%, 90%, 91%, 92%, 93%, 94%, 95%, 96%, 97%, 98% or 99% identity with the amino acid sequence selected from SEQ ID NO: 10 or 28; or

(ii) comprises or consists of an amino acid sequence selected from SEQ ID NO: 10 or 28; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 10 or 28.

In some specific embodiments of the invention, the bispecific antibody of the invention comprises the first antigen-binding domain and the second antigen-binding domain, wherein

the first antigen-binding domain specifically binds CLDN18.2, and the second antigen-binding domain specifically binds CD3. The first antigen-binding domain contains three complementary determining regions A1-HCDR1, A1-HCDR2 and A1-HCDR3 contained in A1-VH as shown in SEQ ID NO: 4, and three complementary determining regions A1-LCDR1, A1-LCDR2 and A1-LCDR3 contained in VL as shown in SEQ ID NO: 9;

the second antigen-binding domain comprises three complementary determining regions A2-HCDR1, A2-HCDR2 and A2-HCDR3 contained in A2-VH as shown in SEQ ID NO: 22, 30 or 32, and three complementary determining regions A2-LCDR1, A2-LCDR2 and A2-LCDR3 contained in A2-VL as shown in SEQ ID NO: 27.

In some specific embodiments of the invention, the bispecific antibody of the invention comprises the first antigen-binding domain and the second antigen-binding domain, wherein the first antigen-binding domain specifically binds CLDN18.2, and the second antigen-binding domain specifically binds CD3, wherein

the first antigen-binding domain comprises A1-HCDR1, A1-HCDR2, A1-HCDR3: SEQ ID NO: 1, 2 and 3 as shown in the following amino acid sequence, and A1-LCDR1, A1-LCDR2 and A1-LCDR3: SEQ ID NO: 6, 7 and 8 as shown in the following amino acid sequence, respectively; and

the second antigen-binding domain comprises

(i) A2-HCDR1, A2-HCDR2, A2-HCDR3 as shown in the following amino acid sequences: SEQ ID NO: 19, 20 and 21, and LCDR1, LCDR2 and LCDR3 as shown in the following amino acid sequences: SEQ ID NO: 24, 25 and 26, respectively; or

(ii) A2-HCDR1, A2-HCDR2, A2-HCDR3 as shown in the following amino acid sequences: SEQ ID NO: 19, 20 and 29, and LCDR1, LCDR2 and LCDR3 as shown in the following amino acid sequences: SEQ ID NO: 24, 25 and 26, respectively; or

(iii) A2-HCDR1, A2-HCDR2, A2-HCDR3 as shown in the following amino acid sequences: SEQ ID NO: 19, 31 and 21, and LCDR1, LCDR2 and LCDR3 as shown in the following amino acid sequences: SEQ ID NO: 24, 25 and 26, respectively.

In some specific embodiments of the invention, the bispecific antibody of the invention comprises the first antigen-binding domain and the second antigen-binding domain, wherein the first antigen-binding domain specifically binds CLDN18.2, and the second antigen-binding domain specifically binds CD3, wherein

the first antigen-binding domain comprises A1-VH comprising or consisting of the amino acid sequence shown in SEQ ID NO: 4 or an amino acid sequence having at least 90%, 91%, 92%, 93%, 94%, 95%, 96%, 97%, 98% or 99% identity with the amino acid sequence; and A1-VL comprising or consisting of the amino acid sequence shown in SEQ ID NO: 9 or an amino acid sequence having at least 90% identity with the amino acid sequence;

the second antigen-binding domain comprises A2-VH comprising or consisting of the amino acid sequence shown in SEQ ID NO: 22, 30 or 32 or an amino acid sequence having at least 90% identity with the amino acid sequence; and A2-VL comprising or consisting of the amino acid sequence shown in SEQ ID NO: 27 or an amino acid sequence having at least 90%, 91%, 92%, 93%, 94%, 95%, 96%, 97%, 98% or 99% identity with the amino acid sequence.

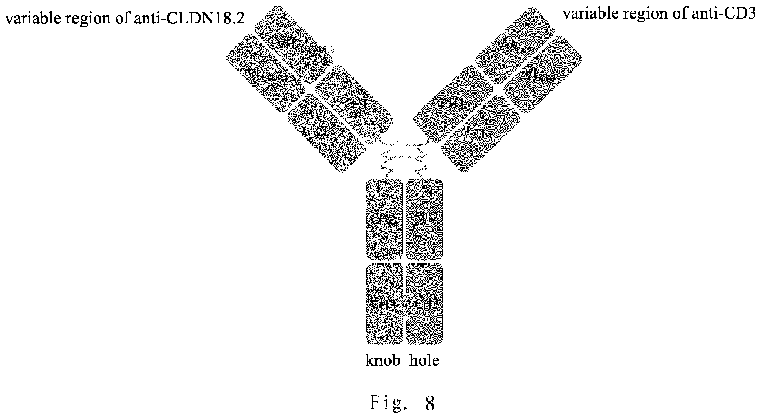

In some specific embodiments of the invention, the bispecific antibody of the invention is the bispecific antibody of IgG-like structure, that is, it comprises the part of the heavy chain and the light chain binding to CLDN18.2, and the part of the heavy chain and the light chain of binding to CD3. In some embodiments, there is a Knob-into-Hole sequence in the CH3 region of the two heavy chains (Shane Atwell et al., Journal of Molecular Biology, 1997; A. Margaret Merchant et al., Nature Biotechnology, 1998), thus forming a knob-in-hole structure. In some embodiments, the structure of the bispecific antibody of the invention is shown in Figure 8.

In some embodiments, the part of the heavy chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 37;

(ii) comprises or consists of an amino acid sequence selected from SEQ ID NO: 37; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 37.

In some embodiments, the part of the light chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 38;

(ii) comprises or consists of an amino acid sequence selected from SEQ ID NO: 38; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 38.

In some embodiments, the part of the heavy chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 41, 39 or 42;

(ii) comprises or consists of an amino acid sequence selected from SEQ ID NO: 41, 39 or 42; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 41, 39 or 42.

In some embodiments, the part of the light chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 40; or

(ii) comprises or consists of an amino acid sequence selected from SEQ ID NO: 40; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 40.

In one embodiment of the invention, the amino acid change described herein includes the substitution, insertion or deletion of amino acids. Preferably, the amino acid change described herein is an amino acid substitution, preferably a conservative substitution.

In a preferred embodiment, the amino acid change described in the invention occurs in a region outside the CDR (for example, in FR). More preferably, the amino acid change described in the invention occurs in the region outside the heavy chain variable region and/or the light chain variable region. In some embodiments, the amino acid change described in the invention occurs in the Fc region of the constant region of the heavy chain of the antibody. In the preferred embodiment, the amino acid change in the Fc region weakens or deletes the ADCC and/or CDC function of the antibody.

In some embodiments, the substitution is a conservative substitution. The conservative substitution refers to the replacement of an amino acid by another amino acid in the same category. For example, one acidic amino acid is replaced by another acidic amino acid, one basic amino acid is replaced by another basic amino acid, or one neutral amino acid is replaced by another neutral amino acid. Exemplary substitution is shown in the following table:

| Original residue | Exemplary substitution | Preferred conservative amino acid substitution |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| I1e (I) | Leu, Val, Met, Ala, Phe, N-leucine | Leu |
| Leu (L) | N-leucine, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, N-leucine | Leu |

In some embodiments, the substitution occurs in the CDR region of the antibody. Generally, the obtained variant has modification (for example, improvement) and/or will have some biological characteristics that are basically retained by the parent antibody in terms of some biological characteristics (for example, increased affinity) relative to the parent antibody. An example substitution variant is an affinity mature antibody.

In some embodiments, one or more amino acid modifications can be introduced into the Fc region of the antibody provided herein to produce Fc region variants to change one or more functional characteristics of the antibody, such as serum half-life, complement binding, complement dependent cytotoxicity, Fc receptor binding, and/or antibody dependent cytotoxicity. Fc region variants may include human Fc region sequences (such as human IgG1, IgG2, IgG3, or IgG4 Fc region) that comprise amino acid changes (such as substitutions) at one or more amino acid positions.

In some embodiments, it may be necessary to produce antibodies modified by cysteine engineering, such as "thioMAb", in which one or more residues of the antibody are substituted by cysteine residues.

In some embodiments, the antibody provided herein may be further modified to comprise other non-protein components known in the art and readily available. Parts suitable for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, dextran, polyvinyl alcohol, polyvinylpyrrolidone, poly-1,3-diane, poly-1,3,6-triane, ethylene/maleic anhydride copolymer, polyamino acid (homopolymer or random copolymer), and dextran or poly(n-vinylpyrrolidone)polyethylene glycol, propylene glycol homopolymer, polyethylene oxide/ethylene oxide copolymer, polyoxyethylated polyol (such as glycerin), polyvinyl alcohol, and mixtures thereof.

In some embodiments, in the bispecific antibody of the invention, the first antigen-binding domain that specifically bind to CLDN18.2 or the combination of heavy chain and light chain is fully human, and the second antigen-binding domain that bind to CD3 or the combination of heavy chain and light chain is humanized.

**III. The nucleic acid of the invention and the host cell comprising the same**

In one aspect, the invention provides nucleic acids encoding variable regions or heavy or light chains of any of the above bispecific antibodies. In one embodiment, a vector comprising the nucleic acid is provided. In one embodiment, the vector is an expression vector, such as pcDNA3.1. In one embodiment, a host cell comprising the nucleic acid or the vector is provided. In one embodiment, the host cell is eukaryotic. In another embodiment, the host cell is selected from yeast cells, mammalian cells (such as CHO cells (such as CHO-S) or 293 cells (such as 293F or HEK293 cells) or other cells suitable for the preparation of antibodies or fragments thereof. In another embodiment, the host cell is prokaryotic.

In one aspect, the nucleic acid of the invention may comprise a nucleic acid encoding the amino acid sequence of the light chain variable region and/or the heavy chain variable region of the antibody, or a nucleic acid encoding the amino acid sequence of the light chain and/or the heavy chain of the antibody.

For example, the nucleic acid of the invention comprises the nucleic acid encoding the amino acid sequence selected from any of SEQ ID NO: 4, 9, 22, 27, 30, 32, 37-42, or encoding the amino acid sequence having at least 85%, 90%, 91%, 92%, 93%, 94%, 95%, 96%, 97%, 98% or 99% identity with the amino acid sequence selected from any of SEQ ID NO: 4, 9, 22, 27, 30, 32, 37-42.

The invention also covers nucleic acids that hybridize with the following nucleic acids under strict conditions or have one or more substitutions (such as conservative substitutions), deletions or insertions compared to the following nucleic acids: nucleic acids comprising nucleic acid sequences encoding amino acid sequences selected from shown in any of SEQ ID NO: 4, 9, 22, 27, 30, 32, 37-42; or a nucleic acid comprising nucleic acid sequences encoding amino acid sequences having at least 85%, 90%, 91%, 92%, 93%, 94%, 95%, 96%, 97%, 98% or 99% identity with the amino acid sequence shown in any of SEQ ID NO: 4, 9, 22, 27, 30, 32, 37-42.

In one embodiment, one or more vectors comprising the nucleic acid are provided. In one embodiment, the vector is an expression vector, such as an eukaryotic expression vector. The vectors include but are not limited to viruses, plasmids, cosmid, λ Phage or yeast artificial chromosome (YAC). In one embodiment, the vector is pcDNA3.1.

In one embodiment, a host cell comprising the vector is provided. Suitable host cells for cloning or expressing vectors encoding antibodies include prokaryotic or eukaryotic cells described herein. For example, antibodies can be produced in bacteria, especially when glycosylation and Fc effector functions are not required. After expression, the antibody can be separated from the bacterial cell paste in the soluble fraction and can be further purified.

In one embodiment, the host cell is eukaryotic. In another embodiment, the host cell is selected from yeast cells, mammalian cells or other cells suitable for preparing antibodies or fragments thereof. For example, eukaryotic microorganisms such as filamentous fungi or yeast are suitable hosts of cloning or expression for vectors encoding antibodies. For example, fungi and yeast strains whose glycosylation pathway has been "humanized" lead to the production of antibodies with partial or complete human glycosylation patterns. Host cells suitable for expressing glycosylation antibodies are also derived from multicellular organisms (invertebrates and vertebrates). Vertebrate cells can also be used as hosts. For example, mammalian cell lines adapted to suspension growth can be used. Other examples of useful mammalian host cell lines are monkey kidney CV1 line (COS-7) transformed with SV40; human embryonic kidney system (HEK293, 293F or 293T cells), etc. Other useful mammalian host cell lines include Chinese hamster ovary (CHO) cells, including DHFR-CHO cells, CHO-S cells, ExpiCHO, etc.; and myeloma cell lines such as Y0, NS0 and Sp2/0. A mammalian host cell line suitable for producing antibodies is known in the art.

## IV. Production and purification of antibody molecule of the invention

In one embodiment, a method for preparing the antibody molecule of the invention is provided, wherein the method includes culturing a host cell comprising nucleic acid encoding the antibody (such as any of one polypeptide chain and/or more polypeptide chains) or an expression vector comprising the nucleic acid under conditions suitable for antibody expression, as provided above, and optionally recovering the antibody from the host cell (or host cell culture medium).

In order to recombine and produce the antibody molecule of the invention, the nucleic acid encoding the antibody (such as the antibody described above, such as any of one polypeptide chain and/or multiple polypeptide chains) is separated and inserted into one or more vectors for further cloning and/or expression in the host cell. Such nucleic acids can be easily separated and sequenced using conventional procedures (for example, by using oligonucleotide probes that can specifically bind to genes encoding the heavy and light chains of antibodies).

The antibody molecules prepared as described herein can be purified by known existing technologies such as high-performance liquid chromatography, ion exchange chromatography, gel electrophoresis, affinity chromatography, size exclusion chromatography, etc. The actual conditions used to purify specific proteins also depend on factors such as net charge, hydrophobicity, hydrophilicity, etc., which are obvious to those skilled in the art. The purity of the antibody molecule of the invention can be determined by any of a variety of well-known analytical methods, which include size exclusion chromatography, gel electrophoresis, high-performance liquid chromatography, etc.

## V. Assays

The antibody provided herein can be identified, screened, or characterized by its physical/chemical properties and/or biological activity through a variety of assays known in the art. On the one hand, the antigen-binding activity of the antibody of the invention is tested, for example, by known methods such as ELISA, Western blotting, etc. The methods known in the art can be used to determine the binding to CLDN18.2 and/or CD3 and an exemplary method is disclosed herein. In some embodiments, radioimmunoassay (RIA) or biomembrane thin-layer interferometry or MSD or surface plasmon resonance (SPR) or flow cytometry are used.

On the other hand, competitive assay can be used to identify antibodies that compete with any bispecific antibody disclosed herein for binding to CLDN18.2 and/or CD3. In some embodiments, such competitive antibodies bind to the same or overlapping epitopes (such as linear or conformational epitopes) as the bispecific antibodies disclosed herein.

The invention also provides an assay for identifying antibodies with biological activity. The biological activities can include, for example, binding to CLDN18.2 and/or CD3 (for example, binding to human CLDN18.2 and/or CD3), binding to cells expressing CLDN18.2 and/or CD3, activation of T cells, stimulation of secretion of cytokines, inhibition and killing of tumor cells, etc. Antibodies with such biological activity in vivo and/or in vitro are also provided.

In some embodiments, the antibody of the invention is tested for such biological activity.

The cells used for any of the above in vitro assays include cell lines which naturally express CLDN18.2 and/or CD3, or express or overexpress CLDN18.2 and/or CD3 through modification. Such cells also include cell lines that express CLDN18.2 and/or CD3 and those that do not normally express CLDN18.2 and/or CD3 and transfected by coding DNA. In some embodiments, such cells are gastric cancer cells or pancreatic cancer cells. In some embodiments, these cells are CHO cells expressing CLDN18.2. In some embodiments, these cells are cell lines NUGC-4, KATO III and DAN-G, such as KATO III and DAN-G cell lines that overexpress CLDN18.2. In some embodiments, such cells are T cells, such as human T lymphocytes, such as Jurkat cells.

It can be understood that the combination of the antibody of the invention and other active agents can be used for any of the above determination methods.

## VI. Pharmaceutical composition

In some embodiments, the invention provides comprising any antibody or composition thereof, preferably the composition is a pharmaceutical composition. In one embodiment, the composition further comprises pharmaceutically acceptable supplementary material. In one embodiment, a composition, for example, a pharmaceutical composition, comprises a combination of an antibody or a fragment thereof of the invention, and one or more other therapeutic agents.

The invention further includes a bispecific antibody of the invention or a composition comprising the same (including a pharmaceutical composition). These compositions may further comprise suitable pharmaceutically acceptable supplementary material, such as pharmaceutically acceptable carriers and pharmaceutically acceptable excipients including buffers known in the art.

As used herein, "pharmaceutically acceptable carrier" includes any and all physiologically compatible solvents, dispersion media, isotonic agents and absorption retardants.

For the use and use of pharmaceutically acceptable supplementary material, see also "Handbook of Pharmaceutical Excipients", 8th edition, R.C. Rowe, P.J. Seskey and S.C. Owen, Pharmaceutical Press, London, Chicago.

The composition of the invention can be in various forms. These forms include, for example, liquid, semi-solid and solid dosage forms, such as liquid solution (for example, injectable solution and infusion solution), powder or suspension, liposome and suppository. The preferred form depends on the intended mode of administration and therapeutic use.

A drug comprising the antibody described herein can be prepared by mixing the antibody of the invention with the required purity with one or more optional pharmaceutically acceptable supplementary material, preferably in the form of lyophilized formulation or aqueous solution.

The pharmaceutical composition or formulation of the invention can further comprise more than one active ingredients, which are required for the specific indication to be treated, preferably those active ingredients with complementary activities that will not adversely affect each other. For example, it is ideal to also provide other therapeutic agents, such as chemotherapeutic agents, angiogenesis inhibitors, cytokines, cytotoxic agents, other antibodies, small molecular drugs or immunomodulators (such as immune checkpoint inhibitors or agonists). The said active ingredients are appropriately combined in an effective amount for the intended use.

Sustained release formations can be prepared. Suitable examples of sustained release formulations include semi-permeable matrices comprising solid hydrophobic polymers containing antibodies, and the said matrices are in the form of shaped articles, such as films or microcapsules.

## VII. Pharmaceutical combination and kit

In some embodiments, the invention further provides pharmaceutical combinations or pharmaceutical combination products, which include the antibodies of the invention, and one or more other therapeutic agents (such as therapeutic agents, including chemotherapy agents, angiogenesis inhibitors, cytokines, cytotoxic agents, other antibodies, small molecule drugs or immunomodulators (such as immune checkpoint inhibitors or agonists) etc.).

Another object of the invention is to provide a kit comprising the pharmaceutical combination of the invention, preferably in the form of drug dose unit. Therefore, the dose unit can be provided according to the regimen or interval of the administration.

In one embodiment, the kit of the invention comprises:

- a first container comprising a pharmaceutical composition containing the bispecific antibody of the invention;

- a second container comprising a pharmaceutical composition containing other therapeutic agents.

## VIII. Use and Method

On the one hand, the invention provides a method for preventing or treating tumors (such as cancer) in a subject, including administering to the subject the therapeutically effective anti-CLDN18.2 and anti-CD3 bispecific antibody or fragment thereof (preferably antigen-binding fragments), the pharmaceutical composition, the pharmaceutical combination or the kit of the invention.

In some embodiments, the patients with tumor (such as cancer) have CLDN18.2 (such as elevated levels, such as nucleic acid or protein levels).

In some embodiments, the tumors, such as cancer, include solid tumors, blood tumors and metastatic lesions. In one embodiment, examples of solid tumors include malignant tumors. Cancer can be in the early, middle or late stage or metastatic cancer.

In some embodiments, the tumor treatment will benefit from the inhibition of CLDN18.2 at nucleic acid or protein levels.

In a specific embodiment, the antibody of the invention can kill tumor cells, and/or inhibit the proliferation of tumor cells, such as tumor cells expressing CLDN18.2, such as gastric cancer cells or pancreatic cancer cells.

In another specific embodiment, the anti-CLDN18.2 x CD3 bispecific antibody of the invention can activate T cells.

Therefore, the antibody of the invention is applicable to prevent or treat any tumor or cancer in which the effector mechanism of cytotoxic T cells is required, or any tumor or cancer requiring T cell recruitment.

In some embodiments, tumor is at immune escape of tumor.

In some embodiments, the tumor is a cancer, such as gastric cancer or pancreatic cancer, and the subject can be a mammal, such as a primate, preferably a higher primate, such as a human (for example, an individual suffering from the disease described herein or at risk of suffering from the disease described herein). In one embodiment, the subject suffers from the disease described herein (for example, cancer) or is at risk of suffering from the disease described herein. In some embodiments, the subjects are receiving or have received other treatments, such as chemotherapy and/or radiotherapy. In some embodiments, the subjects have received immunotherapy before or are receiving immunotherapy.

In other aspects, the invention provides the use of the antibody molecule or pharmaceutical composition or pharmaceutical combinations or kits in the production or preparation of a medicine, which are used for the purposes described herein, for example, for the prevention or treatment of related diseases or disorders mentioned herein.

In some embodiments, the antibody molecule or pharmaceutical composition or pharmaceutical combination or kit of the invention may delay the onset of the disease and/or symptoms related to the disease.

In some embodiments, the antibody molecule or pharmaceutical composition of the invention can also be used in combination with one or more other therapies, such as therapeutic modes and/or other therapeutic agent(s), for the uses described herein, such as for the prevention and/or treatment of related diseases or disorders mentioned herein.

In some embodiments, the therapeutic modes include surgery, radiotherapy, local irradiation or focused irradiation, etc.

In some embodiments, the therapeutic agents are selected from chemotherapeutic agents, angiogenesis inhibitors, cytokines, cytotoxic agents, other antibodies, small molecule drugs or immunomodulators (such as immune checkpoint inhibitors or agonists).

Examples of immunomodulators include immunosuppressants or anti-inflammatory agents. In some embodiments, the immunomodulators also include immune checkpoint inhibitors or agonists. Other exemplary antibodies include antibodies that specifically bind to immune checkpoints.

In some embodiments, the antibody combination described herein can be administered separately, for example, as separate antibodies.

Such combination therapy covers combination administration (for example, two or more therapeutic agents are included in the same or separate formulation), and separate administration. In this case, the administration of the antibody of the invention can occur before, at the same time, and/or after the administration of other therapeutic agents and/or drugs.

The route of administration of the pharmaceutical composition is based on known methods, such as oral, intravenous injection, intraperitoneal, intracerebral (parenchymal), intraventricular, intramuscular, ophthalmic, intra-arterial, intra-portal or intrafocal route; by continuous release system or by implantable device. In some embodiments, the composition may be administered by bolus injection or by continuous infusion or by an implant device.

The composition can also be applied locally via an implanted membrane, sponge or another suitable material on which the required molecules are absorbed or encapsulated. In some embodiments, when an implant device is used, the device can be implanted into any suitable tissue or organ, and the required molecules can be delivered through diffusion, timed release of bolus, or continuous administration.

### IX. Methods and compositions for diagnosis and detection

In some embodiments, any antibody provided herein can be used to detect the presence of CLDN18.2 in biological samples. The term "detection" as used herein includes quantitative or qualitative detection, and exemplary detections may involve immunohistochemistry, immunocytochemistry, flow cytometry (e.g., FACS), magnetic beads complexed with antibody molecules, ELISA, and PCR techniques (e.g., RT-PCR). In some embodiments, the biological sample is blood, serum, or other fluid sample of biological source. In certain embodiments, the biological sample includes cells or tissues. In some embodiments, the biological sample is derived from a proliferative or cancerous lesion related lesion.

In one embodiment, a bispecific antibody is provided for use in a diagnostic or detection method. In another aspect, a method for detecting the presence of CLDN18.2 in a biological sample is provided. In certain embodiments, the method comprises detecting the presence of CLDN18.2 proteins in a biological sample. In certain embodiments, the CLDN18.2 is human CLDN18.2. In certain embodiments, the CD3 is human CD3. In certain embodiments, the method comprises contacting the biological sample with the antibody as described herein in a condition that allows the antibody to bind to CLDN18.2, and detecting whether a complex is formed between the antibody and CLDN18.2. The formation of the complex indicates the presence of CLDN18.2. The method may be an in-vitro or in-vivo method. In one embodiment, the antibody of the invention is used to select a subject suitable for treatment with the bispecific antibody, e.g., wherein CLDN18.2 is a biomarker for selecting the subject.

In one embodiment, the antibody of the invention can be used to diagnose tumors, such as cancers, e.g., to assess (e.g., monitor) the treatment or progression, diagnosis and/or staging of a disease described herein in a subject. In certain embodiments, a labeled bispecific antibody is provided. The label includes, but is not limited to, a label or moiety (e.g., a fluorescent label, a chromophore label, an electron-dense label, a chemoluminescent label, and a radioactive label) that is detected directly, as well as a moiety that is detected indirectly, such as an enzyme or a ligand, for example, by an enzymatic reaction or a molecular interaction.

In some embodiments provided herein, the sample is obtained prior to treatment with the antibody of the invention. In some embodiments, the sample is obtained prior to treatment with other therapies. In some embodiments, the sample is obtained during or after treatment with other therapies.

In some embodiments, the sample is formalin-fixed and paraffin-coated (FFPE). In some embodiments, samples are biopsies (such as core biopsies), surgical specimens (such as specimens from surgical resection), or fine needle aspirates.

In some embodiments, CLDN18.2 is detected prior to treatment, e.g., prior to initial treatment or prior to a treatment after an interval from a certain treatment.

In some embodiments, a method for treating the diseases of the invention is provided, which comprises: detecting the presence of CLDN18.2 in a subject (for example, a sample)(e.g., a sample from the subject), thereby determining a CLDN18.2 value; comparing the CLDN18.2 value to a control value; and if the CLDN18.2 value is greater than the control value, administering a therapeutically effective amount of the bispecific antibody of the invention, optionally in combination with one or more of other therapies, to the subject, thereby treating the said diseases.

These and other aspects and embodiments of the invention are described in the drawings (brief description of the drawings follows) and in the following detailed description of the invention and are illustrated in the following examples. Any or all of the features discussed above and throughout the application may be combined in various embodiments of the invention. The following examples further illustrate the invention. However, it is to be understood that the examples are described by way of illustration and not limitation, and various modifications may be made by those skilled in the art.

## Examples

### Example 1. Construction of stable cell line

#### Preparation of cell line overexpressing human CLDN18.2

A stable cell line expressing human Claudin 18.2 (CLDN18.2 for short, the same below) was constructed using Freedom® CHO-S® Kit (Invitrogen, A1369601) according to the manufacturer's instructions. Firstly, the full-length gene of human CLDN18.2 (UniProt ID: P56856-2) was constructed into the vector pCHO1.0 to form a plasmid. The constructed plasmid was transferred into CHO-S cells (Invitrogen, A1369601) and HEK293 cells (Invitrogen, A14527) by chemical transfection and electric transfection, respectively. After transfection, the cells were subjected to two rounds of pressure screening to obtain cell pools (pools) expressing CLDN18.2 respectively. Then, flow cytometry (MoFlo XDP, Beckman Coulter) was used to isolate the cells with high expression of CLDN18.2. Monoclonal cell lines CHO-hCLDN18.2 and HEK293-hCLDN18.2 stably expressing CLDN18.2 were obtained by dilution method.

#### Preparation of cell line overexpressing human CLDN18.1

A stable cell line expressing human Claudin 18.1 (CLDN18.1 for short, the same below) was constructed using Freedom® CHO-S® Kit (Invitrogen, A1369601) according to the manufacturer's instructions. Firstly, the full-length gene of human CLDN18.1 (UniProt ID: P56856-1) was constructed into the vector pCHO1.0 (Invitrogen, A1369601) to form a plasmid. The constructed plasmid was transferred into CHO-S cells (Invitrogen, A1369601) by chemical transfection. After transfection, the cells were subjected to two rounds of pressure screening to obtain cell pools (pools) expressing CLDN18.1 respectively. Then, flow cytometry (MoFlo XDP, Beckman Coulter) was used to isolate the cells with high expression of CLDN18.1. Monoclonal cell lines CHO-hCLDN18.1 stably expressing CLDN18.1 were obtained by dilution method.

#### Construction of tumor cell line overexpressing CLDN18.2

The full-length gene of human CLDN18.2 (UniProt ID: P56856-2) was constructed into the vector pWPT-GFP (Addgene, 12255) to replace the GFP sequence therein, and the lentivirus packaging vectors psPAX2 (Addgene, 12260) and pMD2. G (Addgene, 12259) were co-transfected into HEK293T (ATCC, CRL-3216) cells for virus packaging. The culture supernatant was collected after 48 hours and 72 hours of culture, and lentivirus was concentrated with PEG8000. The concentrated virus was used to transfect pancreatic cancer DAN-G cells and gastric cancer KATO III cells, and then flow cytometry (MoFlo XDP, Beckman Coulter) was used to select the cells expressing CLDN18.2, to obtain tumor cell lines DAN-G-hCLDN18.2 and KATO III-hCLDN18.2 stably transfected with CLDN18.2.

## Example 2. Production of CLDN18.2 Monoclone

The invention adopted hybridoma technology and immunizes H2L2 full-human antibody transgenic mice (purchased from Harbor BioMed) with cells (CHO-hCLDN18.2) obtained in Example 1. Then the spleen cells of mice were obtained and electrofused with myeloma cells. After that, the supernatant was collected and hybridoma cells specifically expressing anti-CLDN18.2 antibody were screened by flow cytometry (FACS), and the secreted antibody did not bind to CLDN18.1. The cells to be tested (HEK293-hCLDN18.2) obtained in Example 1 were counted and diluted to $1 \times 10^6$ cells/ml, and were added 100 $\mu$l/well to the U-shaped bottom 96-well plate. It was centrifuged at 500 g for 5 min, and the cell culture medium was discarded. The culture supernatant of hybridoma 96-well plate was added to the U-shaped plate 100 $\mu$l/well and the cells were resuspended, and the plate was let stand on ice for 30min. The supernatant was removed at 500g for 5min, and the cells was washed with PBS once. 100 $\mu$l of FITC-labeled secondary antibody against mouse Fab (1:500 diluted in PBS) was added to each well, and 100 $\mu$l of FITC-labeled secondary antibody against human Fab was added to the positive control antibody. It was incubated in dark on ice for 30 minutes, the supernatant was removed at 500g for 5min, and the cells was washed with PBS once. 50 $\mu$l 1 $\times$ PBS for resuspending cells was used and FACS for detection was used on machine. The positive clones were re-screened with CHO-hCLDN18.1 using the same method as above. After two rounds of screening, the whole human antibody clone HB37A6 was obtained.

## Example 3. Preparation of recombinant CLDN18.2 monoclonal antibody

The anti-CLDN18.2 monoclonal antibody HB37A6 (see CN202010570517.X) and the control antibody zolbetuximab (abbreviated as Zmab, sequence from INN117) were expressed in HEK293 cells (Invitrogen, A14527) as forms of full-length monoclonal antibodies.

Firstly, the expression vector was constructed, and the heavy chain variable region and light chain variable region (see sequence information) of HB37A6 and control antibody were placed at the N-terminal of the heavy chain constant region (SEQ ID NO: 5) and light chain kappa constant region (SEQ ID NO: 10) of human IgG1 respectively. After that, it was constructed into pcDNA3.1 expression vector with N-terminal signal peptide to obtain a light and heavy chain expression vector. The obtained light and heavy chain expression vectors were co-transfected into HEK293 cells through PEI (Polysciences Inc, 23966), and the culture medium supernatant was collected after 7 days of culture. The supernatant was purified by Protein A column (Hitrap Mabselect Sure, GE 11-0034-95), and then ultrafiltration was performed and the liquid was changed into PBS (Gibco, 70011-044). The concentration was detected by A280 method and the purity was determined by SEC-HPLC method. The antibody solution with purity greater than 95% was obtained, and the recombinant CLDN18.2 monoclonal antibody HB37A6 was obtained.

The specific transfection and purification process is as follows:
Expi293 cells (Invitrogen, A14527) were passaged according to the required transfection volume, and the cell density was adjusted to $1.5 \times 10^6$ cells/ml the day before transfection. The cell density was about $3 \times 10^6$ cells/ml on the day of transfection. 1/10 of the final volume of Opti-MEM medium (Gibco, 31985-070) was taken as the transfection buffer and appropriate plasmid was added according to 1.0$\mu$g/ml transfected cells. Appropriate polyethylene imines (PEI) (23966) were added into the plasmid (the ratio of plasmid to PEI was 1:3 in 293F cells), mixed well and incubated at room temperature for 20 min to obtain the DNA/PEI mixture. The DNA/PEI mixture was added into the cell slowly, the flask was shook gently while adding, and then cultured in an incubator with 36.5 °C, 8% $CO_2$. The cell material was obtained after seven days and the cell supernatant was collected for purification.

The Protein A column (Hitrap Mabselect Sure, GE, 11-0034-95) for purification was treated with 0.1 M NaOH for 2 hours, and the glass bottles were washed with distilled water and dried at 180 °C for 4 hours. The collected cell material was centrifuged at 4500 rpm for 30 min before purification, and then filtering the supernatant using 0.22$\mu$M filter. 10 column volumes of bound buffer (sodium phosphate 20mM. NaCl 150M, PH7.0) was used to balance Protein A column. The filtered supernatant was added into the purification column and balanced with 10 column volumes of binding buffer. 5 ml of eluent buffer (citric acid + sodium citrate 0.1M, pH3.5) was added before collecting the eluent, and then 80$\mu$L 2M Tris-HCl was added to eluent per ml. The collected antibody was concentrated and exchanged into PBS (Gibco, 70011-044) by ultrafiltration, before the concentration was detected.

## Example 4. Determination of affinity of CLDN18.2 antibody by SPR method

The equilibrium dissociation constant ($K_D$) of HB37A6 binding to human CLDN18.2 was determined by surface plasma resonance (SPR). Human Claudin 18.2 (GenScrip, P50251802) was coupled to the surface of CM5 chip (GE Healthcare, 29-1496-03) using an amino-coupling kit (GE Healthcare, BR-1006-33) according to the manufacturer's instructions. After coupling, 1 M ethanolamine was injected to seal the remaining activation sites. The binding and dissociation between the chip surface antigen and the antibody in the mobile phase were detected by Biocore (GE Healthcare, T200) according to the manufacturer's instructions to obtain the affinity and kinetic constants. The serially diluted antibody (0-100 nM) flowed through the chip surface in order from low concentration to high concentration, with binding time of 180 s and dissociation time of 600 s. Finally, 10 mM Glycine pH 1.5 (GE Healthcare, BR-1003-54) was used to regenerate the chip. The resulting data were analyzed using the Biocore T200 analysis software and the dynamic analysis was conducted using the 1:1 combination model. As shown in Table 1, the affinity of HB37A6 was superior than that of control antibody Zmab.

Table 1. Affinity constant (equilibrium dissociation constant) of CLDN18.2 antibody detected by SPR method

| Antibody | ka (1/Ms) | kd (1/s) | KD (M) |
|---|---|---|---|
| Zmab | 3.482E+5 | 7.894E-4 | 2.267E-9 |
| HB37A6 | 4.252E+5 | 3.063E-4 | 7.203E-10 |

## Example 5. Binding specificity of CLDN18.2 antibody to CLDN18 cells

The binding of the above anti-CLDN18.2 monoclonal antibody HB37A6 and the control antibody Zmab to the CHO-S cell line stably transfected with human CLDN18.2 and human CLDN18.1 obtained in Example 1 (i.e. CHO-hCLDN18.2 and CHO-hCLDN18.1 prepared as described in Example 1) was determined by flow cytometry (FACS).

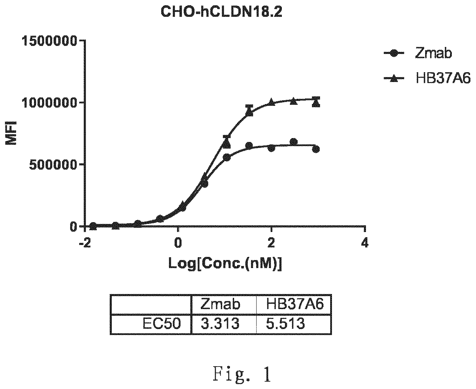

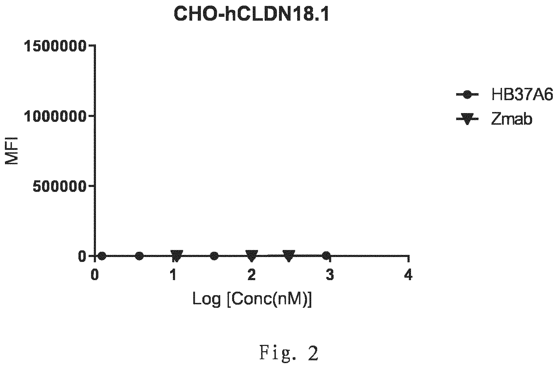

Specifically, the cells to be tested (CHO-hCLDN18.2 and CHO-hCLDN18.1) obtained in Example 1 were counted and diluted to $1 \times 10^6$ cells/ml, added to the U-shaped bottom 96-well plate at 100 $\mu$l/well, and then centrifuged at 500 g for 5 min before the cell culture medium was removed. The anti-CLDN18.2 monoclonal antibody HB37A6 and the control antibody Zmab were added to the U-shaped plate followed by resuspending the cells at 100$\mu$l/well. The initial concentration of the antibody was 900 nM, and it was diluted three times serially, a total of 10 concentration points before leaving it on ice for 30min, and removing the supernatant at 500g for 5min, and washing the cells with PBS once before adding goat anti-human Fc PE-labeled secondary antibody (Southern Biotech, J2815-5H87B) at 100 $\mu$l/well, and incubating in dark on ice for 30 min, removing the supernatant at 500g for 5min, and washing the cells with PBS once. 50 $\mu$l $1 \times$ PBS was used to resuspend cells and FACS was used for detection. GraphPad Prism software was used to analyze the experimental data and get Figure 1 and Figure 2. As shown in Figure 1 and Figure 2, the antibodies specifically bind to human CLDN18.2, but not to human CLDN18.1.

## Example 6. Binding of CLDN18.2 antibody to tumor cell line

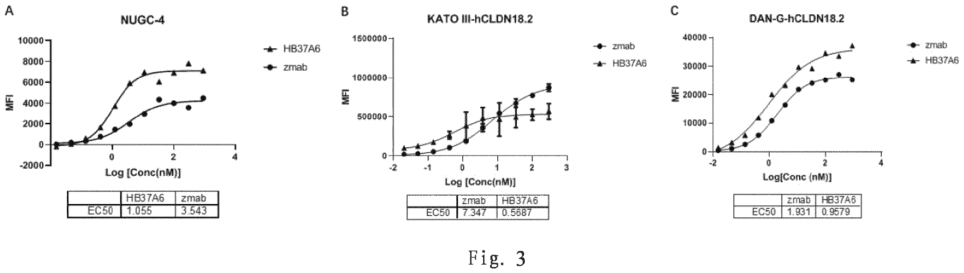

Referring to Example 5, the binding of HB37A6 to gastric cancer cell line NUGC-4 (JCRB cell bank, JCRB0834), gastric cancer cell line KATO III-hCLDN18.2 and pancreatic cancer cell line DAN-G-hCLDN18.2 was determined by FACS. Figure 3 showed that the whole human antibody HB37A6 had superior tumor cell-specific binding than the control antibody Zmab.

## Example 7. Antitumor effect of CLDN18.2 antibody in vivo

1. The activity of antibody to DAN-G-CLDN18.2 tumor bearing mouse model

HB37A6 antibody was used to test its anti-tumor effect in NOD-SCID mice bearing human pancreatic cancer (female NOD-SCID mice (15-18g) were purchased from Beijing Vital River Laboratory Animal Technology Co., Ltd, n=60). The human pancreatic cancer cell DAN-G-hCLDN18.2 constructed in Example 1 was routinely passaged for subsequent in vivo experiments, and then centrifuged to collect the cells and dispersed DAN-G-hCLDN18.2 with PBS ($1\times$) to obtain cell density of $12 \times 10 \char`^ 5$/ml suspension. The cell suspension was mixed with matrigel gel at 1:1 to prepare cells with a concentration of $6 \times 10 \char`^ 5$ cells/ml cell suspension. On day 0, 0.2 ml of cell suspension was subcutaneously inoculated into the right abdominal region of NOD-SCID mice to establish the DAN-G-CLDN18.2 tumor-bearing mice model.

The tumor volume of each mouse was detected after 5 days of the inoculation of tumor cells. The mice with tumor volume ranging from 43.36 mm$^3$ to 89.47 mm$^3$ were picked and divided into serpentine groups according to the size of tumor (8 mice in each group).

Each mouse was administered with hIgG (Equitech-Bio, batch number 160308-02), HB37A6 and control antibody Zmab at a dose of 10mg/kg each time, the administration was on the 5th, 9th, 12th and 16th day after inoculation respectively, and the tumor volume of mice was monitored 2-3 times a week. Tumor volume measurement: the maximum long axis (L) and the maximum wide axis (W) of the tumor were measured with a vernier caliper. The tumor volume was calculated according to the following formula: $V=L\times W^2/2$. Electronic balance was used to measure body weight.

2. The activity of antibody on NUCG-4 tumor bearing mice model

HB37A6 antibody was selected to test its anti-tumor effect in NOG mice bearing human gastric cancer (female NOG mice (15-18g) purchased from Beijing Vital River Laboratory Animal Technology Co., Ltd., n=100). PBMC cells (Allcells) were recovered and collected by centrifugation, before dispersing PBMC cells with PBS (1×). The cell density was $2.5 \times 10 ^ 6$ cells/ml cell suspension, and 0.2 ml cell suspension was taken on the 0th day for intravenous injection into the eyes of NOG mice to establish a NOG humanized mouse model.

NUGC-4 cells were routinely recovered and passaged for subsequent in vivo experiments. NUGC-4 cells were centrifuged and then dispersed with PBS (1×) into cell density of $12 \times 10 ^ 6$ cells/ml, followed by mixed with matrigel gel at 1:1 to prepare cells with a concentration of $6 \times 10 ^ 6$ cells/ml cell suspension. On the 5th day, 0.2 ml of cell suspension was subcutaneously inoculated into the right abdominal region of NOG humanized mice to establish NUCG-4 tumor-bearing mice model.

On the first day after the inoculation of tumor cells, mice were randomly divided into 7 mice/group. Each mouse was administered with hIgG (Equitech-Bio, batch number 160308-02), HB37A6 and control antibody Zmab at a dose of 10mg/kg each time, on the 1st, 5th, 8th and 12th days after the inoculation. The tumor volume and weight of mice were monitored 2-3 times a week. Tumor volume measurement: the maximum long axis (L) and the maximum wide axis (W) of the tumor are measured with a vernier caliper. The tumor volume was calculated according to the following formula: $V=L \times W^2/2$. Electronic balance was used to measure body weight. The relative tumor inhibition rate (TGI%) was calculated on the 26th day of inoculation with the following formula:

$$TGI\%=100\% * \text{ (tumor volume of control group – tumor volume of treatment group)/ (tumor volume of control group – tumor volume of control group before administration)}.$$

3. Results

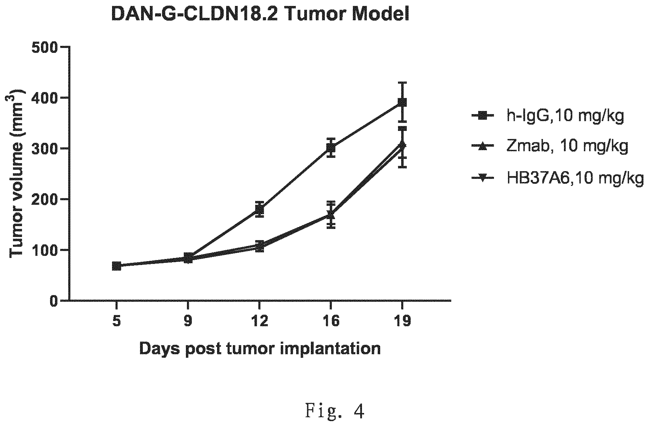

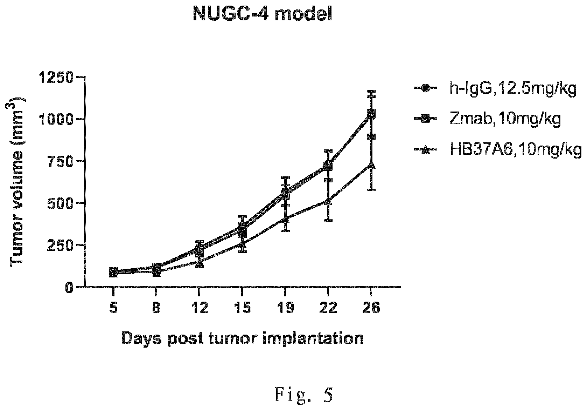

As shown in Figure 4, both HB37A6 and the control antibody Zmab can inhibit tumor growth in the human pancreatic cancer DAN-G-CLDN18.2 mouse model, with the TGI of HB37A6 being 28% and that of Zmab being 24%. As shown in Figure 5, HB37A6 showed superior anti-tumor effect than the control antibody Zmab on the human gastric cancer NUGC-4 mouse model with the TGI of HB37A6 being 31%, and that of Zmab being 0%.

**Example 8 Preparation of anti-CD3 monoclonal antibody**

The murine CD3 antibody sp34 (U.S. Pat. No. 8236308; J. Immunol. Methods., 1994, 178:195) and the anti-CD3 monoclonal antibodies HzSP34.24, HzSP34.87, HzSP34.97 from humanization and affinity optimization of sp34 were expressed in HEK293 cells (Invitrogen, A14527) as full-length monoclonal antibodies.

The heavy chain variable region, heavy chain variable region and light chain variable region (see sequence information) of HzSP34.24, HzSP34.87, HzSP34.97 and sp34 were placed at the N-terminal of the IgG1 heavy chain constant region (SEQ ID NO: 23) and light chain lambda constant region (SEQ ID NO: 28). After that, they were constructed into pcDNA3.1 expression vector with N-terminal signal peptide to obtain light and heavy chain expression vectors. The obtained light and heavy chain expression vectors were co-transfected into HEK293 cells through PEI (Polysciences Inc, 23966), and the culture medium supernatant was collected after 7 days of culture. The supernatant was purified by Protein A column (Hitrap Mabselect Sure, GE 11-0034-95), and then ultrafiltration was performed and the liquid was changed into PBS (Gibco, 70011-044). The concentration was detected by A280 method and the purity was determined by SEC-HPLC method. The antibody solution with purity greater than 95% was obtained, and the recombinant anti-CD3 monoclonal antibodies HzSP34.24, HzSP34.87, HzSP34.97 and mouse CD3 antibody sp34 were obtained. See Example 3 for the specific transfection and purification process.

**Example 9. Binding of anti-CD3 monoclonal antibody to human CD3**

Jurkat cells were immortalized human T lymphocytes, which expressed human CD3 complex. The cell line was used to detect the binding of the antibody of invention and the cell.

The detailed operation was as follows: inoculating Jurkat cells (Promega, J1621) into U-shaped 96-well plate at $2\times 10^5$/well, adding the antibodies to be detected (anti-CD3 monoclonal antibodies HzSP34.24, HzSP34.87, HzSP34.97 and mouse CD3 antibody sp34) to the corresponding cell wells according to a series of concentration gradients (the initial concentration of antibody molecules is 500 nM, 3 folds serial dilution), incubating at 4 °C for 30 minutes, and then using PBS to wash the unbound portion, and adding the PE fluorescent secondary antibody (SouthernBiotech, J2815-5H87B) of sheep anti-human Fc. The flow cytometry (FACSCELESTA, BD) was used for on-line detection after incubation at 4 °C for 15 min.

Results:

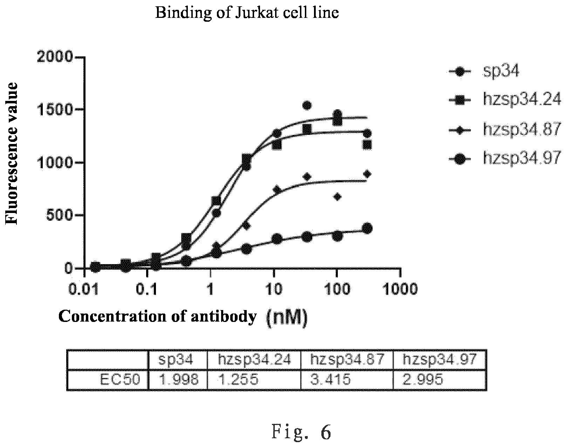

As shown in Figure 6, the humanized CD3 antibody HzSP34.24 and mouse antibody sp34 showed comparable affinity at the cellular level, while the affinity of Hzsp34.87 and Hzsp34.97 with CD3 at the cellular level was weakened to varying degrees.

**Example 10. Detection of T cell activation function of CD3 antibody**

The invention used Jurkat NFAT (Nuclear Factor of Activated T) reporter cell (Promega, J1621) to detect the T cell activation function of anti-CD3 monoclonal antibodies HzSP34.24, HzSP34.87, HzSP34.97 and murine CD3 antibody sp34. The cell was an engineered Jurkat T cell. When the cell was activated through TCR-CD3 pathway, it released luciferase substrate into the experimental system through the downstream signal NFAT, whereby to detect the activation of T cells.

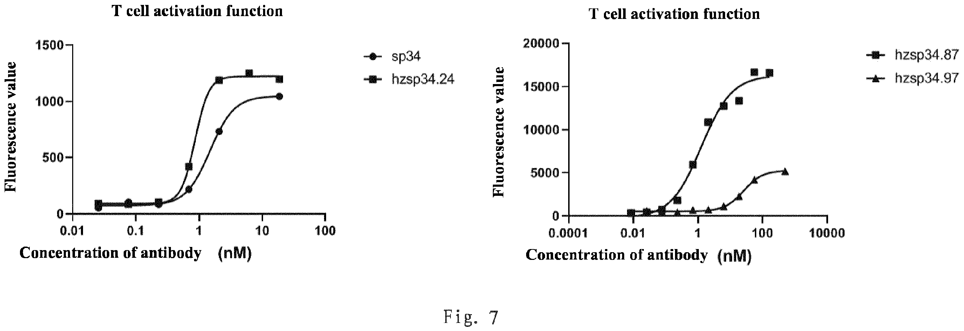

The detailed operation of this example was to use a white 96-hole flat plate, and $4 \times 10^4$ Jurkat NFAT cells were mixed with various antibody molecules of corresponding concentration (the initial concentration of antibody molecules was 500 nM, 3 folds serial dilution) in each well, incubated in incubator 37 °C for 6-8 hours before addition of 100 μL Bio-Glo (Promega, G7940) to each well, and wavelength detection was performed using a Microplate Reader (Spectra, Molecular Devices). Results was shown in Figure 7, mouse CD3 antibody sp34, humanized CD3 antibody HzSP34.24, Hzsp34.87 and Hzsp34.97 all have the ability to activate T cells.

**Example 11. Construction and preparation of bispecific antibody**

According to the combinations in Table 2, the sequence of antigen-binding region of anti-CLDN18.2 monoclonal antibody HB37A6 and three different anti-human CD3 monoclonal antibodies HzSP34.24, HzSP34.87 and HzSP34.97 were respectively used to construct the bispecific antibodies 030, 032 and 033 in "1+1" form to target CLDN18.2 × CD3, and their structures were shown in Figure 8. Specifically, the sequence of the heavy chain variable region specifically targeting the antigen-binding domain of CLDN18.2 was SEQ ID NO: 4, and the sequence of the light chain variable region was SEQ ID NO: 9; the sequences of heavy chain variable region of antigen-binding domain specifically targeting CD3 are SEQ ID NO: 22, SEQ ID NO: 30 and SEQ ID NO: 32, and the sequence of light chain variable region was SEQ ID NO: 27. The Fc segment of the antibody selects the IgG1 LALA sequence of knob-in-hole structure (A. Margaret Merchant et al., Nature Biotechnology, 1998). Therefore, the heavy chain of CLDN18.2 moiety of the bispecific antibody was SEQ ID NO: 37, and the light chain was SEQ ID NO: 38. The heavy chain of CD3 moiety of bispecific antibody was SEQ ID NO: 39, 41 and 42, and the light chain was SEQ ID NO: 40.

The process of plasmid construction of bispecific antibody was as follows: the heavy chain sequence of CLDN18.2 (SEQ ID NO: 37), the light chain sequence of CLDN18.2 (SEQ ID NO: 38), the heavy chain sequence of CD3 (SEQ ID NO: 39, 41 and 42), and the light chain sequence of CD3 (SEQ ID NO: 40) were inserted into the vector pcDNA3.1 (Invitrogen, V790-20) to obtain the heavy chain plasmid, light chain plasmid at the CLDN18.2 moiety , and the heavy chain plasmid and light chain plasmid at the CD3 moiety. Then PEI (Polysciences, 23966) was used to transiently transfect the heavy chain plasmids, light chain plasmids of CLDN18.2 moiety and heavy chain plasmids and light chain plasmids of CD3 moiety into Expi293 cells (Invitrogen, A14527) to express three antibody molecules of CLDN18.2 moiety and CD3 moiety. After 7 days, the cell fermentation broth was obtained, filtered and clarified, and captured with the Protein A column (GE Healthcare, 11-0034-95) of Hitrap Mabselect Sure, respectively, to obtain antibodies at the moieties for CLDN18.2 and CD3. After the concentration was detected by A280 method, both antibody moieties were mixed in a ratio of 1:1, and then adding an appropriate amount of reducing agent GSH before reacting overnight at room temperature, followed by removing the reducing agent by ultrafiltration to terminate the reaction. After that, MonoS cation exchange chromatography (GE Healthcare, 17-5168-01) was used for fine purification. Liquid A was 20 mM sodium phosphate buffer (pH 6.6), and liquid B was 20 mM sodium phosphate buffer (pH 6.6) containing 1M sodium chloride. The elution gradient was 0-50% (30 column volume). The eluted protein solution was ultrafiltration and transferred to PBS (Gibco, 70011-044). The molecular weight was determined by mass spectrometry and the purity was identified by SEC-HPLC. The obtained 030, 032 and 033 bispecific antibodies were used in the following examples.

Table 2. List of CD3/CLDN18.2 bispecific antibodies

|  | anti-CLDN18.2 moiety | anti-CD3 moiety |
|---|---|---|
| 030 | HB37A6 | HzSP34.24 |
| 032 | HB37A6 | HzSP34.87 |
| 033 | HB37A6 | HzSP34.97 |

**Example 12. Determination of affinity of bispecific antibodies**

The equilibrium dissociation constant (KD) of the bispecific antibody of the invention binding to human CD3 protein was determined by the Biolayer Interferometry (BLI). The affinity determination of BLI method was carried out according to the existing methods (Estep, P et al., High throughput solution based measurement of antibody-antigen affinity and affinity binding. MAbs, 2013.5 (2): 270-8).

An appropriate number of AHC (18-5060, Fortebio) sensors was taken and soaked in SD buffer (1x PBS, BSA 0.1%, Tween-20 0.05%) according to the number of samples at half an hour before the experiment. 100 $\mu$l of SD buffer, each bispecific antibody and human CD3 protein (CT026H0323H, Sino Biological Inc.) were added into 96-well black polystyrene semi-quantitative micro-well plate (Greiner, 675076). Fortebio Octet Red96 was used for detection, and the sensor position was selected according to the sample position. The setting parameters of the instrument were as follows: operation steps: Baseline, Loading~1 nm, Baseline, Association and Dissociation; the operation time of each step depended on the speed of sample combination and dissociation. The rotational speed was 1000 rpm and the temperature was 30 °C. ForteBio Octet analysis software was used to analyze KD value.

The affinity of bispecific antibodies was shown in Table 3. The affinity of CD3 moiety of 030 molecule was the highest, with 7.4nM. The affinity of CD3 moiety of 032 and 033 molecules decreased in turn, with 89nM and 440nM respectively.

Table 3. Affinity of CD3 moiety of the bispecific antibodies

|  | Ka(1/Ms) | Kd(1/s) | KD(M) |
|---|---|---|---|
| 030 | 6.927E+5 | 0.005164 | 7.455E-9 |
| 032 | 8.066E+5 | 0.07183 | 8.906E-8 |
| 033 | 3.788E+5 | 0.1689 | 4.459E-7 |

The equilibrium dissociation constant (KD) of binding to human CLDN18.2 was determined by surface plasma resonance (SPR). According to the manufacturer's instructions, the antigen human Claudin 18.2 (GenScrip, P50251802) was coupled to the surface of CM5 chip (GE Healthcare, 29-1496-03) using the amino-coupling kit (GE Healthcare, BR-1006-33). After coupling, 1 M ethanolamine was injected to block the remaining activation sites. According to the manufacturer's instructions, the binding and dissociation between the chip surface antigen and various bispecific antibodies in the mobile phase were detected by Biocore (GE Healthcare, T200) to obtain the affinity and kinetic constants. The antibody after serial dilution (0-100 nM, twice dilution) flowed through the chip surface in order from low concentration to high concentration, with binding time of 180 s and dissociation time of 600 s. Finally, 10 mM Glycine pH 1.5 (GE Healthcare, BR-1003-54) was used to regenerate the chip. The resulting data were analyzed using the Biocore T200 analysis software and the 1:1 combination model. Results was shown in Table 4, the same clone HB37A6 was used for 030, 032 and 033 bispecific antibodies at the CLDN18.2 moiety, and the affinity of the CLDN18.2 moiety was consistent, with a very strong affinity of 0.57nM.

Table 4. Affinity of CLDN18.2 moiety of the bispecific antibodies

|  | Ka(1/Ms) | Kd(1/s) | $K_D$(M) |
|---|---|---|---|
| 030 |  |  |  |
| 032 | 3.47E+05 | 2.00E-04 | 5.76E-10 |
| 033 |  |  |  |

**Example 13. T cell killing test *In vitro***

The 030, 032 and 033 bispecific antibodies obtained in Example 11 were used for T cell killing test *in vitro*. Human peripheral blood mononuclear cells (PBMC, Allcells or Saily) were re-suspended with complete medium RPMI-1640 (Hyclone, SH30809.01)+10% fetal bovine serum (FBS, Hyclone, SH30084.03), and PBMC was adjusted to $2 \times 10^6$ /ml.

NUGC-4 or DAN-G tumor target cell DAN-G-hCLDN18.2 overexpressing Claudin 18.2, non-target cell L363 (DSMZ, ACC49) were labeled with Far-Red (Invitrogen) for 10 min, washed twice and then resuspended in the complete medium, and then the cell concentration was adjusted to $2 \times 10^5$ /ml.

PBMC was mixed with bispecific antibodies 030, 032 and 033 respectively (the initial concentration of 030 and 032 were 1 nM, and the initial concentration of 033 was 400 nM. All antibodies were diluted five times, a total of 10 concentration points), incubated at 37 °C for 30 minutes, and then 50 μL tumor target cells ($1 \times 10^4$) were added to 50 μL PBMC effector cells with 10:1 ratio of effector cells: target cells, followed by incubation at 37 °C for 24 hours, centrifugation, resuspending of the cells with the final concentration of 10 μg/ml of propidium iodide (PI, Invitrogen), and then flow cytometry (BD, FACSCELESTA) was used to detect both Far-Red and PI positive cells. The killing ratio of tumor target cells was calculated by FACSDiva software (BD, Celestsa).

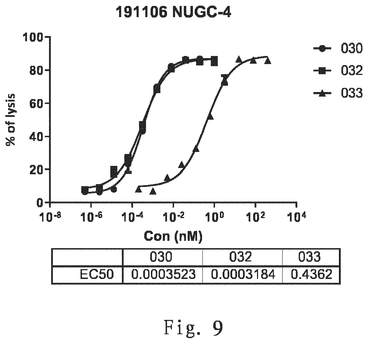

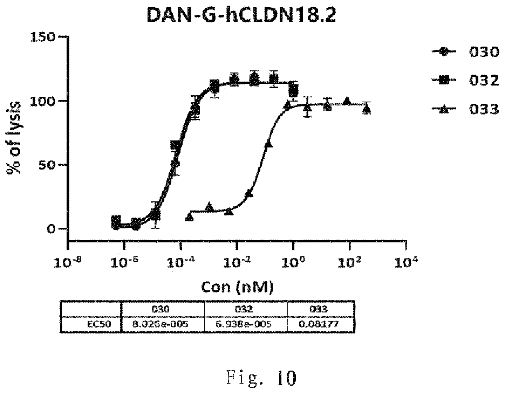

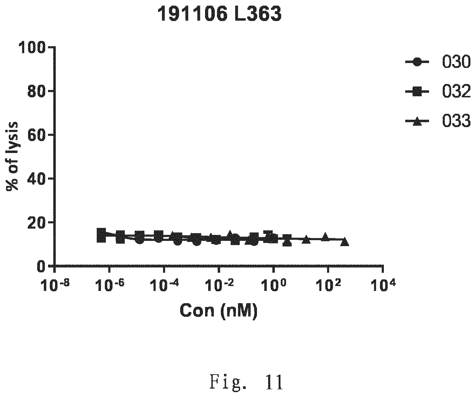

The results in Figure 9 showed that 030 and 032 molecules had very strong killing activity on the gastric cancer cell NUGC-4, and the EC50 value was less than 1pM. The results in Figure 10 showed that the EC50 values for both cells were even less than 0.1pM for DAN-G-hCLDN18.2, a pancreatic cancer cell with high expression of CLDN18.2. The killing activity of 033 molecule for the two tumor cell lines was weaker, lower than 030 and 032 about 1000 times, but it still reached the maximum killing (nearly 100% lysed cells). However, in the case of negative expression of CLDN18.2, 030, 032 and 033 molecules all did not have non-specific killing effect (Fig. 11). This indicates that 030, 032 and 033 molecules all exhibit the specific killing of tumor cells that depends on the expression of CLDN18.2. Moreover, the killing effect of the bispecific antibody of the invention is related to the abundance of CLDN18.2 on the cell surface. Within a certain range of expression abundance, the higher the expression of CLDN18.2 on the cell surface, the better the killing effect is.

**Example 14. Release test of cytokine in vitro**

Human peripheral blood mononuclear cells (PBMC, Allcells or Saily) were resuspended with complete medium RPMI-1640 (Hyclone, SH30809.01)+10% fetal bovine serum (FBS, Hyclone, SH30084.03), and PBMCs were adjusted to $2 \times 10^6$/ml. The concentrations of NUGC-4 or DAN-G tumor target cell DAN-G-hCLDN18.2 overexpressing Claudin 18.2 were adjusted to $2 \times 10^5$/ml.

PBMCs were mixed with bispecific antibodies 030, 032 and 033 respectively, incubated at 37 °C for 30 min, and then 50 μL Tumor target cells ($1 \times 10^4$) were added to 50 μL PBMC effector cells with 10:1 ratio of effector cells: target cells, followed by incubation at 37°C for 24 hours, centrifugation, and obtaining cell supernatant. Human Th1/Th2/Th17 Kit (BD, article No. 560484) was used to detect cytokines followed by incubation at room temperature for 3 hours and detection by flow cytometry (BD, FACSCELESTA), and then the release of cytokines in the supernatant was analyzed by FCAP Array software (BD).

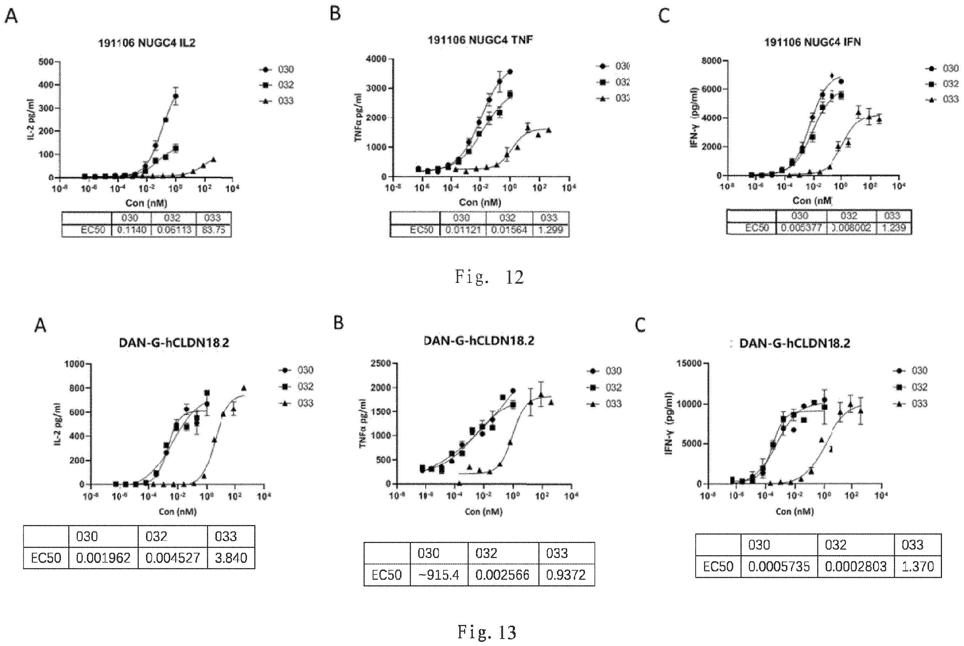

The results in Figure 12 and Figure 13 showed 030, 032 and 033 molecules was able to mediate the high release of IL-2, TNFα and IFNgamma cytokines on gastric cancer cell NUGC-4 and pancreatic cancer cell DAN-G-hCLDN18.2, which were positively correlated with the affinity of CD3 moiety.

**Example 15. T cell activation test *in vitro***

Human peripheral blood mononuclear cells (PBMC, Allcells or Saily) were re-suspended with complete medium RPMI-1640 (Hyclone, SH30809.01)+10% fetal bovine serum (FBS, Hyclone, SH30084.03), and PBMCs were adjusted to $2 \times 10^6$/ml.

The concentration of NUGC-4 or DAN-G tumor target cell DAN-G-CLDN18.2 overexpressing Claudin 18.2 were adjusted to $2 \times 10^5$/ml. PBMC was mixed with bispecific antibodies 030, 032 and 033 respectively, incubated at 37 °C for 30 min, and then 50 μL tumor target cells ($1 \times 10^4$) were added to 50 μL PBMC effector cells with 10:1 ratio of effector cells: target cells, followed by incubation at 37 °C for 24 hours, centrifugation, removing the supernatant, and then the cells were incubated with BV421 anti-human CD3 (Biolegend, 317344), PerCP/Cy5.5 mouse anti-human CD4 (BD, 552838), APC/Cy7 anti-human CD8a (Biolegend, 300926), PE anti-human CD25 (Biolegend, 302606), FITC anti-human CD69 (Biolegend, 310904) for 1 hour at 4 °C, and then washed three times with $1 \times$ PBS, and the ratio of both CD25 and CD69 positive cells in CD4+and CD8+T cells was detected by flow cytometry (BD, FACSCELESTA). The ratio of both CD25 and CD69 positive cells in CD4+and CD8+T cells was calculated by FACSDiva software (BD, Celestsa), which was the ratio of CD4 and CD8+T cells in activation.

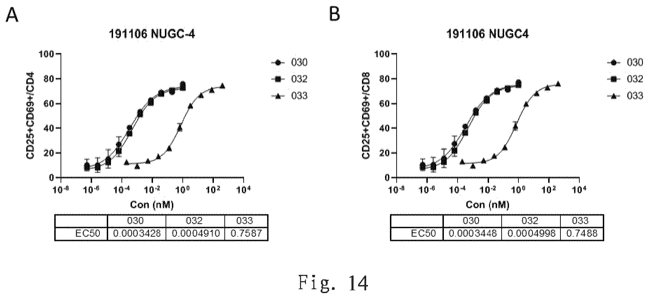

As shown in Figure 14, 030, 032 and 033 molecules could specifically activate T cells in the co-culture of gastric cancer cell NUGC-4, and the activation ability was positively correlated with the affinity of CD3 moiety.

**Example 16. Pharmacodynamic experiment in vivo - gastric cancer model**

In this experiment, the anti-tumor effect of bispecific antibody on NUGC-4 tumor was studied in NOG female mice. 49 female NOG mice (Beijing Vital River Laboratory Animal Technology Co., Ltd.) were selected.

PBMC cells (Allcells) were resuscitated and centrifuged. PBS ($1\times$) was used to disperse PBMC cells to obtain the cell suspension with a cell density of $2\times10^7$/ml. 200 $\mu$L cell suspension was taken to inject PBMC cells into the orbital vein of mice, $4\times10^6$/mouse.

NUGC-4 cells were routinely resuscitated and subcultured for subsequent experiments *in vivo.* cells were centrifuged and collected, NUGC-4 cells were dispersed with PBS ($1\times$), and the cells with cell density of $6 \times 10^7$ cells/ml were mixed with matrigel gel at 1:1 to prepare cell suspension with a cell density of $3 \times 10^7$/ml. On the third day, 0.2 ml of cell suspension was subcutaneously inoculated into the right abdominal region of NOG humanized mice to establish a mice model bearing NUCG-4 tumor.

On the 7th day after cell inoculation, the maximum wide axis and the maximum long axis of the tumor in mouse were measured with a vernier caliper, and the tumor volume were calculated. The mice with tumor volume between 53.35 mm$^3$ and 168.07 mm$^3$ were picked, and the mice were divided into serpentine group according to the tumor volume (6 mice in each group). The bispecific antibodies 030, 032 and 033 of the invention and the negative control h-IgG (Equitech-Bio, batch number 160308-02) were injected intravenously into each mouse, with the dose of 0.3 mg/kg and 1 mg/kg, once a week, a total of 4 times, and the frequency of measuring tumor volume was twice a week. Meanwhile, the tumor inhibition rate (TGI%) was calculated as follows:

$$\text{TGI\%} = 100\% * (\text{tumor volume of control group - tumor volume of treatment group})/(\text{tumor volume of control group - tumor volume of control group before administration}).$$

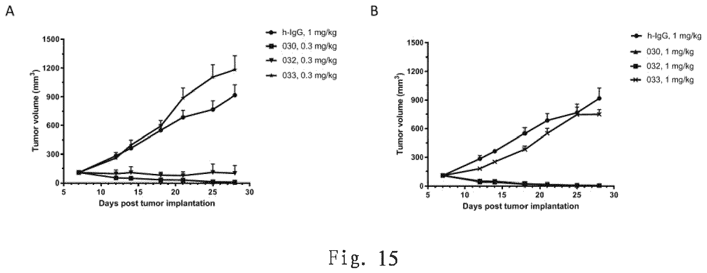

As shown in Figure 15, in the mouse model bearing human gastric cancer NUCG-4 tumor, 030 and 032 can reach 100% TGI at a low dose of 0.3mg/kg, and reach 50% CR (complete remission) at a high dose of 1mg/kg (3 of 6 mice have achieved complete tumor vanishment). However, 033 molecule almost had no effect at low dose, and TGI can reach 20% at a dose of 1mg/kg. During the whole experiment, the weight of mice in the experimental group and the control group did not drop.

**Example 17. Pharmacodynamic experiment in vivo - pancreatic cancer model**

In this experiment, the anti-tumor effect of bispecific antibody on DAN-G-Claudin 18.2 tumor was studied in NOG female mice. PBMC cells were injected into the orbital vein in 49 NOG mice (Beijing Vital River Laboratory Animal Technology Co., Ltd.), $4\times10^6$/mouse, and inoculation volume was 200ul/mouse (as shown in Example 18). This was recorded as day 0.

The human pancreatic cancer cell DAN-G-CLDN18.2 constructed in Example 1 was routinely sub-cultured for subsequent in vivo experiments. The cells were centrifuged and collected. DAN-G-CLDN18.2 were dispersed with PBS ($1\times$) to obtain the suspension with cell density of $10\times10^6$/ml. The cell suspension was mixed with matrigel gel at 1:1 to prepare the cell suspension with a concentration of $5\times10^6$ cells/ml. On day 0, 0.2 ml of cell suspension was taken and subcutaneously injected into the right abdominal region of NOD-SCID mice to establish a humanized model of DNA-G pancreatic cancer with CLDN18.2 overexpression.

7 days after the inoculation of tumor cells, the maximum wide axis and the maximum long axis of the tumor were measured with a vernier caliper, and the tumor volume were calculated. The mice with tumor volume in the range of 46.42 mm$^3$~120.64 mm$^3$ were divided into serpentine group according to the tumor size (6 mice in each group).

The bispecific antibodies 030, 032 and 033 of the invention and the negative control h-IgG (Equitech-Bio, batch number 160308-02) were injected intravenously into each mouse, with the intraperitoneal dose of 0.3 mg/kg and 1 mg/kg, once a week, a total of 4 times, and the frequency of measuring tumor volume was twice a week. Meanwhile, the tumor inhibition rate (TGI%) was calculated as follows:

$$\text{TGI\%} = 100\% * (\text{tumor volume of control group - tumor volume of treatment group})/ (\text{tumor volume of control group - tumor volume of control group before administration}).$$

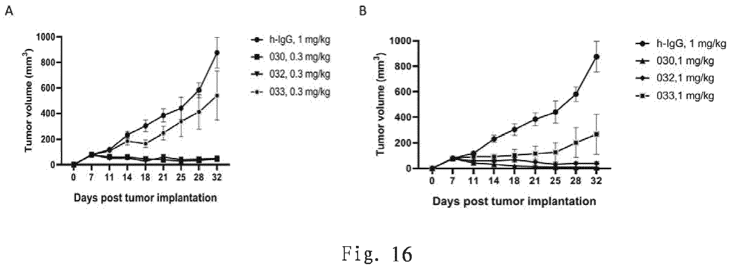

As shown in Figure 16, in the humanized model of DNA-G pancreatic cancer with CLDN18.2 overexpression, 030 and 032 molecules can reach 100% TGI at both doses. The 033 molecule also reached 42% TGI at 0.3mg/kg of and 76% TGI at 1mg/kg respectively, which may be related to the high expression of CLDN18.2 in DAN-G-CLDN18.2 pancreatic cancer cells. During the whole experiment, the weight of mice in the experimental group and the control group did not drop.

**Example 18. PK experiment in mouse**

In this study, female Balb/C mice (Vitoliva) were injected with 10 mg/kg of 030, 032 and 033 via tail vein to study their pharmacokinetics in mice. After administration, blood was taken from the eyes of mice at 0.086hr, 0.5hr, 2hr, 6hr, 24hr, 48hr, 4day, 7day, 14day and 21day respectively, and the blood was centrifuged at 4 °C at 3000rpm for 10 min to collect serum. The antibody content in serum was determined by ELISA, and the half-lives of 030, 032 and 033 in mice were calculated.

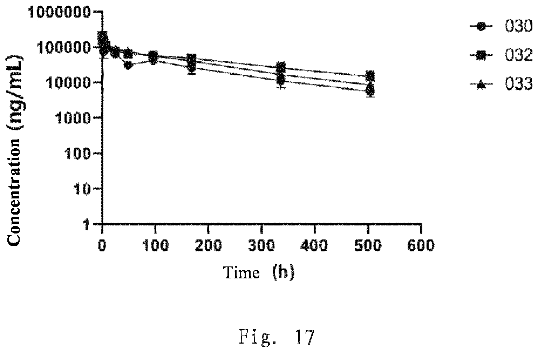

The experimental results were shown in Figure 17. The half-lives of 030, 032 and 033 in mice were similar to PK of normal monoclonal antibodies. It further showed that the bispecific antibody constructed by the invention did not affect the half-life of the antibody.

Sequence information:

| SEQ ID NO | Name of antibodies | HB37A6 |
|---|---|---|
| 1 | HCDR1 | GFTFSSYVMS |
| 2 | HCDR2 | TISHSGGSTYYADSVKG |
| 3 | HCDR3 | DAPYYDILTGYRY |
| 4 | Variable region of heavy chain (VH) | EVQLLDSGGGLVQPGGSLRLSCAASGFTFSSYVMSWVRQAPGKGLNWVSTISHSGGSTYYADSVKGRFTISRDNSKNTLYLQMNSLRAEDTAVYYCAIDAPYYDILTGYRYWGQGTLVTVSS |
| 5 | Constant region of heavy chain (HC) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKAEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 6 | LCDR1 | RASQSISSWLA |
| 7 | LCDR2 | KASSLES |
| 8 | LCDR3 | QQYNSYSYT |
| 9 | Variable region of light chain (VL) | DIQMTQSPSTLSASVGDRVTITCRASQSISSWLAWYQQKPGKAPKLLIYKASSLESGVPSRFSGSGSGTEFTLTISSLQPDDFATYYCQQYNSYSYTFGQGTKLEIK |
| 10 | Constant region of light chain (LC) | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

| SEQ ID NO | Name of antibodies | Positive control antibody Zmab |
|---|---|---|
| 11 | HCDR1 | GYTFTSYWIN |
| 12 | HCDR2 | NIYPSDSYTNYNQKFK |
| 13 | HCDR3 | SWRGNSFDY |

| SEQ ID NO | Name of antibodies | Positive control antibody Zmab |
|---|---|---|
| 14 | VH | QVQLQQPGAELVRPGASVKLSCKASGYTFTSYWINWVKQRPGQGLEWIGNIYPSDSYTNYNQKFKDKATLTVDKSSSTAYMQLSSPTSEDSAVYYCTRSWRGNSFDYWGQGTTLTVSS |
| 5 | HC | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKAEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQD<br><br>WLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 15 | LCDR1 | KSSQSLLNSGNQKNYLT |
| 16 | LCDR2 | WASTRES |
| 17 | LCDR3 | QNDYSYPFT |
| 18 | VL | DIVMTQSPSSLTVTAGEKVTMSCKSSQSLLNSGNQKNYLTWYQQKPGQPPKLLIYWASTRESGVPDRFTGSGSGTDFTLTISSVQAEDLAVYYCQNDYSYPFTFGSGTKLEIK |
| 10 | LC | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| SEQ ID NO | Name of antibodies | HzSP34.24 |
| 19 | HCDR1 | GFTFNTYAMN |
| 20 | HCDR2 | RIRSKYNNYATYYADSVKD |
| 21 | HCDR3 | HGNFGQSYVSWFAY |
| 22 | VH | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNFGQSYVSWFAYWGQGTTVTVSS |

| SEQ ID NO | Name of antibodies | HzSP34.24 |
|---|---|---|
| 23 | HC | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPGK |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVL |
| 28 | LC | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVE KTVAPTECS |
| SEQ ID NO | Name of antibodies | HzSP34.87 |
| 19 | HCDR1 | GFTFNTYAMN |
| 20 | HCDR2 | RIRSKYNNYATYYADSVKD |
| 29 | HCDR3 | HYNFGQSYVSWFAY |
| 30 | VH | EVQLVESGGGLVQPGGSLKLSCAASGFTFNTYAMNWVRQASGKGLEW VGRIRSKYNNYATYYADSVKDRFTISRDDSKSTLYLQMNSLKTEDTAVY YCARHYNFGQSYVSWFAYWGQGTTVTVSS |

| SEQ ID NO | Name of antibodies | HzSP34.87 |
|---|---|---|
| 23 | HC | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPGK |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVL |
| 28 | LC | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVE KTVAPTECS |
| SEQ ID NO | Name of antibodies | HzSP34.97 |
| 19 | HCDR1 | GFTFNTYAMN |
| 31 | HCDR2 | RIRSKAGGYATYYADSVKD |
| 21 | HCDR3 | HGNFGQSYVSWFAY |
| 32 | VH | EVQLVESGGGLVQPGGSLKLSCAASGFTFNTYAMNWVRQASGKGLEW VGRIRSKAGGYATYYADSVKDRFTISRDDSKSTLYLQMNSLKTEDTAVY YCARHGNFGQSYVSWFAYWGQGTTVTVSS |

| SEQ ID NO | Name of antibodies | HzSP34.97 |
|---|---|---|
| 23 | HC | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPGK |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVL |
| 28 | LC | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVE KTVAPTECS |
| SEQ ID NO | | knobs (CH1+CH2+CH3) |
| 33 | | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSRDELTKN QVSLWCLVKGFYPSDIAVEWESNGQPENNYKTTPPVLDSDGSFFLYSKL TVDKSRWQQGNVFSCSVMHEALHNHYTQKSLSLSPGK |

(continued)

| SEQ ID NO | | holes (CH1+CH2+CH3) |
|---|---|---|
| 34 | | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSRDELTKN QVSLSCAVKGFYPSDIAVEWESNGQPENNYKTTPPVLDSDGSFFLVSKL TVDKSRWQQGNVFSCSVMHEALHNHYTQKSLSLSPGK |
| SEQ ID NO | | sp34antibody |
| 35 | VH | EVQLVESGGGLVQPKGSLKLSCAASGFTFNTYAMNWVRQAPGKGLEW VARIRSKYNNYATYYADSVKDRFTISRDDSQSILYLQMNNLKTEDTAMY YCVRHGNFGNSYVSWFAYWGQGTLVTVSS |
| 23 | HC | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTK NQVSLTCLVKGFYPSDIAVEWESNGQPENNYKTTPPVLDSDGSFFLYSK LTVDKSRWQQGNVFSCSVMHEALHNHYTQKSLSLSPGK |
| 36 | VL | QAVVTQESALTTSPGETVTLTCRSSTGAVTTSNYANWVQEKPDHLFTGL IGGTNKRAPGVPARFSGSLIGDKAALTITGAQTEDEAIYFCALWYSNLW VFGGGTKLTVL |
| 28 | LC | GQPKAAPSVTLFPPSSEELQANKATLVCLISDFYPGAVTVAWKADSSPV KAGVETTTPSKQSNNKYAASSYLSLTPEQWKSHRSYSCQVTHEGSTVE KTVAPTECS |

38

| SEQ ID NO | | CLDN18.2 moiety HB37A6 of the bispecific antibodies |
|---|---|---|
| 37 | Heavy chain | EVQLLDSGGGLVQPGGSLRLSCAASGFTFSSYVMSWVRQAPGKGLNW VSTISHSGGSTYYADSVKGRFTISRDNSKNTLYLQMNSLRAEDTAVYYC AIDAPYYDILTGYRYWGQGTLVTVSSASTKGPSVFPLAPSSKSTSGGTA ALGCLVKDYFPEPVTVSWNSGALTSGVHTFPAVLQSSGLYSLSSVVTVP SSSLGTQTYICNVNHKPSNTKVDKKVEPKSCDKTHTCPPCPAPEAAGGP SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEK TISKAKGQPREPQVYTLPPSRDELTKNQVSLWCLVKGFYPSDIAVEWES NGQPENNYKTTPPVLDSDGSFFLYSKLTVDKSRWQQGNVFSCSVMHEA LHNHYTQKSLSLSPGK |
| 38 | Light chain | DIQMTQSPSTLSASVGDRVTITCRASQSISSWLAWYQQKPGKAPKLLIY KASSLESGVPSRFSGSGSGTEFTLTISSLQPDDFATYYCQQYNSYSYTFG QGTKLEIKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGEC |
| SEQ ID NO | | CD3 moiety HzSP34.24 of the bispecific antibodies |
| 39 | Heavy chain | EVQLVESGGGLVQPGGSLKLSCAASGFTFNTYAMNWVRQASGKGLEW VGRIRSKYNNYATYYADSVKDRFTISRDDSKSTLYLQMNSLKTEDTAVY YCARHGNFGQSYVSWFAYWGQGTTVTVSSASTKGPSVFPLAPSSKSTS GGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALP APIEKTISKAKGQPREPQVYTLPPSRDELTKNQVSLSCAVKGFYPSDIAV EWESNGQPENNYKTTPPVLDSDGSFFLVSKLTVDKSRWQQGNVFSCSV MHEALHNHYTQKSLSLSPGK |

39

(continued)

| SEQ ID NO | | CD3 moiety HzSP34.24 of the bispecific antibodies |
|---|---|---|
| 40 | Light chain | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVLGQPKAAPSVTLFPPSSEELQANKATLVCLISDFYPG AVTVAWKADSSPVKAGVETTTPSKQSNNKYAASSYLSLTPEQWKSHRS YSCQVTHEGSTVEKTVAPTECS |
| SEQ ID NO | | CD3 moiety HzSP34.87 of the bispecific antibodies |
| 41 | Heavy chain | EVQLVESGGGLVQPGGSLKLSCAASGFTFNTYAMNWVRQASGKGLEW VGRIRSKYNNYATYYADSVKDRFTISRDDSKSTLYLQMNSLKTEDTAVY YCARHYNFGQSYVSWFAYWGQGTTVTVSSASTKGPSVFPLAPSSKSTS GGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALP APIEKTISKAKGQPREPQVYTLPPSRDELTKNQVSLSCAVKGFYPSDIAV EWESNGQPENNYKTTPPVLDSDGSFFLVSKLTVDKSRWQQGNVFSCSV MHEALHNHYTQKSLSLSPGK |
| 40 | Light chain | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVLGQPKAAPSVTLFPPSSEELQANKATLVCLISDFYPG AVTVAWKADSSPVKAGVETTTPSKQSNNKYAASSYLSLTPEQWKSHRS YSCQVTHEGSTVEKTVAPTECS |

EP 4 223 778 A1

| SEQ ID NO | | CD3 moiety HzSP34.97 of the bispecific antibodies |
|---|---|---|
| 42 | Heavy chain | EVQLVESGGGLVQPGGSLKLSCAASGFTFNTYAMNWVRQASGKGLEW VGRIRSKAGGYATYYADSVKDRFTISRDDSKSTLYLQMNSLKTEDTAVY YCARHGNFGQSYVSWFAYWGQGTTVTVSSASTKGPSVFPLAPSSKSTS GGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALP APIEKTISKAKGQPREPQVYTLPPSRDELTKNQVSLSCAVKGFYPSDIAV EWESNGQPENNYKTTPPVLDSDGSFFLVSKLTVDKSRWQQGNVFSCSV MHEALHNHYTQKSLSLSPGK |
| 40 | Light chain | QAVVTQEPSLTVSPGGTVTLTCRSSTGAVTTSNYANWVQQKPGQAPRG LIGGTNKRAPGVPARFSGSLLGDKAALTLLGAQPEDEAEYYCALWYSN LWVFGQGTKLTVLGQPKAAPSVTLFPPSSEELQANKATLVCLISDFYPG AVTVAWKADSSPVKAGVETTTPSKQSNNKYAASSYLSLTPEQWKSHRS YSCQVTHEGSTVEKTVAPTECS |

**Claims**

1. A bispecific antibody, which comprises a first antigen-binding domain and a second antigen-binding domain, wherein the first antigen-binding domain specifically binds to CLDN18.2, and the second antigen-binding domain specifically binds to CD3, wherein the first antigen-binding domain comprises three complementary determining regions A1-HCDR1, A1-HCDR2 and A1-HCDR3 contained in A1-VH as shown in SEQ ID NO: 4, and three complementary determining regions A1-LCDR1, A1-LCDR2 and A1-LCDR3 contained in VL as shown in SEQ ID NO: 9; the second antigen-binding domain comprises three complementary determining regions A2-HCDR1, A2-HCDR2 and A2-HCDR3 contained in A2-VH as shown in SEQ ID NO: 30, 22 or 32, and three complementary determining regions A2-LCDR1, A2-LCDR2 and A2-LCDR3 contained in A2-VL as shown in SEQ ID NO: 27.

2. The bispecific antibody of claim 1, wherein

   the first antigen-binding domain comprises A1-HCDR1, A1-HCDR2, A1-HCDR3 as shown in the following amino acid sequence: SEQ ID NO: 1, 2 and 3, respectively, and A1-LCDR1, A1-LCDR2 and A1-LCDR3 as shown in the following amino acid sequence: SEQ ID NO: 6, 7 and 8, respectively; and
   the second antigen-binding domain comprises

   (i) A2-HCDR1, A2-HCDR2, A2-HCDR3 as shown in the following amino acid sequence: SEQ ID NO: 19, 20 and 29, respectively, and LCDR1, LCDR2 and LCDR3 as shown in the following amino acid sequence: SEQ ID NO: 24, 25 and 26, respectively; or
   (ii) A2-HCDR1, A2-HCDR2, A2-HCDR3 as shown in the following amino acid sequence: SEQ ID NO: 19, 20 and 21, respectively, and LCDR1, LCDR2 and LCDR3 as shown in the following amino acid sequence: SEQ ID NO: 24, 25 and 26, respectively; or
   (iii) A2-HCDR1, A2-HCDR2, A2-HCDR3 as shown in the following amino acid sequence: SEQ ID NO: 19, 31 and 21, respectively, and LCDR1, LCDR2 and LCDR3 as shown in the following amino acid sequence: SEQ ID NO: 24, 25 and 26, respectively.

3. The bispecific antibody of claim 1 or 2, the first antigen-binding domain comprises a heavy chain variable region and/or a light chain variable region, wherein the heavy chain variable region

   (i) comprises or consists of an amino acid sequence having at least 90%, 91%, 92%, 93%, 94%, 95%, 96%, 97%, 98% or 99% identity with the amino acid sequence selected from SEQ ID NO: 4; or
   (ii) comprises or consists of an amino acid sequence selected from SEQ ID NO: 4; or
   (iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 4, preferably, the said amino acid changes do not occur in the CDR region; and/or

   the light chain variable region

   (i) comprises or consists of an amino acid sequence having at least 90%, 91%, 92%, 93%, 94%, 95%, 96%, 97%, 98% or 99% identity with the amino acid sequence selected from SEQ ID NO: 9; or
   (ii) comprises or consists of an amino acid sequence selected from SEQ ID NO: 9; or
   (iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 9, preferably, the said amino acid changes do not occur in the CDR region.

4. The antibody of any of claims 1-3, wherein the second antigen-binding domain comprises a heavy chain variable region and/or a light chain variable region, wherein the heavy chain variable region

   (i) comprises or consists of an amino acid sequence having at least 90%, 91%, 92%, 93%, 94%, 95%, 96%, 97%, 98% or 99% identity with the amino acid sequence selected from SEQ ID NO: 30, 22 or 32; or
   (ii) comprises or consists of an amino acid sequence selected from SEQ ID NO: 30, 22 or 32; or
   (iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 30, 22 or 32, preferably, the said amino acid changes do not occur in the CDR region; and/or

the light chain variable region

(i) comprises or consists of an amino acid sequence having at least 90%, 91%, 92%, 93%, 94%, 95%, 96%, 97%, 98% or 99% identity with the amino acid sequence selected from SEQ ID NO: 27; or
(ii) comprises or consists of an amino acid sequence selected from SEQ ID NO: 27; or
(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 27, preferably, the said amino acid changes do not occur in the CDR region.

5. The antibody of any of claims 1-4, further comprises a heavy chain constant region and/or a light chain constant region.

6. The antibody of any of claims 1-5, which is a bispecific antibody with IgG-like structure.

7. The antibody of any of claims 1-6, wherein the heavy chain constant region of the antibody is from IgG1 or IgG2 or IgG3 or IgG4, preferably from IgG1.

8. The antibody of any of claims 1-6, which comprises two heavy chain constant regions, one heavy chain constant region A1-HC is connected with the heavy chain variable region A1-VH of the first antigen domain to form the part of the heavy chain binding to CLDN18.2, and the other heavy chain constant region A2-HC is connected with the heavy chain variable region A2-VH of the second antigen binding domain to form the part of the heavy chain binding to CD3, and comprises two light chain constant regions, one light chain constant region A1-LC is connected with the light chain variable region A1-VL of the first antigen domain to form the part of the light chain binding to CLDN18.2, and the other light chain constant region A2-LC is connected with the light chain variable region A2-VL of the second antigen binding domain to form the part of the light chain binding to CD3.

9. The antibody of claim 8, wherein A1-HC can be the same or different from A2-HC, and/or A1-LC can be the same or different from A2-LC.

10. The antibody of claim 8, wherein A1-VH and A1-VL of the part binding to CLDN18.2 are fully human, and A2-VH and A2-VL of the part binding to CD3 are humanized.

11. The antibody of claim 8, wherein the part of the heavy chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 37;
(ii) comprises or consists of an amino acid sequence selected from SEQ ID NO: 37; or
(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 37; and/or

the part of the light chain binding to CLDN18.2

(i) comprises or consists of an amino acid sequence having at least 85%, 90%, 91%, 92%, 93%, 94%, 95%, 96%, 97%, 98% or 99% identity with the amino acid sequence selected from SEQ ID NO: 38;
(ii) comprises or consists of an amino acid sequence selected from SEQ ID NO: 38; or
(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 38.

12. The antibody of claim 8 or 11, wherein
the part of the heavy chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 41, 39 or 42;

(ii) comprises or consists of an amino acid sequence selected from SEQ ID NO: 41, 39 or 42; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 41, 39 or 42; and/or

the part of the light chain binding to CD3

(i) comprises or consists of an amino acid sequence having at least 85%, 90%, 91%, 92%, 93%, 94%, 95%, 96%, 97%, 98% or 99% identity with the amino acid sequence selected from SEQ ID NO: 40; or

(ii) comprises or consists of an amino acid sequence selected from SEQ ID NO: 40; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 40.

13. An isolated nucleic acid that encodes the light chain variable region or heavy chain variable region, or light chain or heavy chain of the antibody of any of claims 1 to 12.

14. A vector comprising the nucleic acid of claim 13, preferably the said vector is an expression vector.

15. A host cell comprising the nucleic acid of claim 13 or the vector of claim 14, preferably, the said host cell is prokaryotic or eukaryotic cell, more preferably selected from yeast cells, mammalian cells (such as 293 cells or CHO cells, such as CHO-S cells or HEK293 cells) or other cells suitable for preparing antibodies or antigen-binding fragments thereof.

16. A method for preparing an antibody binding to CLDN18.2 or antigen-binding fragment thereof, the said method comprises culturing the host cell of claim 15 under the conditions suitable for expressing the nucleic acid encoding the antibody of any of claim 1 to 12, optionally separating the antibody or antigen-binding fragment thereof, and optionally the said method further comprises recovering the said antibody from the said host cell.

17. A pharmaceutical composition, which comprises the antibody binding to CLDN18.2 or antigen-binding fragment thereof of any of claims 1 to 12, and optionally one or more other therapeutic agents, such as chemotherapeutic agents, angiogenesis inhibitors, cytokines, cytotoxic agents, other antibodies, small molecular drugs or immunomodulators (such as immune checkpoint inhibitors or agonists), and optionally a pharmaceutically acceptable supplementary material.

18. A pharmaceutical combination, which comprises the antibody or antigen-binding fragment thereof of any of claims 1 to 12, and optionally one or more other therapeutic agents, such as chemotherapeutic agents, angiogenesis inhibitors, cytokines, cytotoxic agents, other antibodies, small molecular drugs or immunomodulators (such as immune checkpoint inhibitors or agonists).

19. A method for preventing or treating tumor in a subject, the said method comprises administering to the subject an effective amount of the antibody or antigen-binding fragment thereof of any of claims 1 to 12, or the drug composition of claim 17, or the pharmaceutical combination of claim 18.

20. The method of claim 19, wherein the said tumor is a cancer, preferably, the said cancer has an elevated level (such as nucleic acid or protein level) of CLDN18.2, for example, the said cancer is pancreatic cancer or gastric cancer or gastroesophageal junction cancer.

21. The method of any of claims 19-20, the said method further comprises administering one or more therapies to the patient, such as therapeutic modes and/or other therapeutic agents, preferably the therapeutic modes comprise radiotherapy or surgery, or therapeutic agents comprise chemotherapy agents, angiogenesis inhibitors, cytokines, cytotoxic agents, other antibodies, small molecule drugs or immune modulators (such as immune checkpoint inhibitors or agonists).

Fig. 1

**CHO-hCLDN18.1**

Fig. 2

Fig. 3

DAN-G-CLDN18.2 Tumor Model

Fig. 4

**NUGC-4 model**

Legend:
- ● h-IgG,12.5mg/kg
- ■ Zmab,10mg/kg
- ▲ HB37A6,10mg/kg

Fig. 5

Binding of Jurkat cell line

| | sp34 | hzsp34.24 | hzsp34.87 | hzsp34.97 |
|---|---|---|---|---|
| EC50 | 1.998 | 1.255 | 3.415 | 2.995 |

Fig. 6

Fig. 7

Fig. 8

191106 NUGC-4

| EC50 | 030 | 032 | 033 |
|---|---|---|---|
| | 0.0003523 | 0.0003184 | 0.4362 |

Fig. 9

# DAN-G-hCLDN18.2

| | 030 | 032 | 033 |
|---|---|---|---|
| EC50 | 8.026e-005 | 6.938e-005 | 0.08177 |

Fig. 10

191106 L363

Fig. 11

EP 4 223 778 A1

EP 4 223 778 A1

A

**191106 NUGC4 IL2**

| | 030 | 032 | 033 |
|---|---|---|---|
| EC50 | 0.1140 | 0.06113 | 83.75 |

B

**191106 NUGC4 TNF**

| | 030 | 032 | 033 |
|---|---|---|---|
| EC50 | 0.01121 | 0.01564 | 1.299 |

C

**191106 NUGC4 IFN**

| | 030 | 032 | 033 |
|---|---|---|---|
| EC50 | 0.005377 | 0.008002 | 1.239 |

Fig. 12

A

**DAN-G-hCLDN18.2**

| | 030 | 032 | 033 |
|---|---|---|---|
| EC50 | 0.001962 | 0.004527 | 3.840 |

B

**DAN-G-hCLDN18.2**

| | 030 | 032 | 033 |
|---|---|---|---|
| EC50 | ~915.4 | 0.002566 | 0.9372 |

C

**DAN-G-hCLDN18.2**

| | 030 | 032 | 033 |
|---|---|---|---|
| EC50 | 0.0005735 | 0.0002803 | 1.370 |

Fig. 13

Fig. 14

Fig. 15

Fig. 16

Fig. 17

## INTERNATIONAL SEARCH REPORT

| International application No. |
|---|
| **PCT/CN2021/121286** |

**A. CLASSIFICATION OF SUBJECT MATTER**

C07K 16/28(2006.01)i;  C07K 16/46(2006.01)i;  C12N 15/13(2006.01)i;  C12N 15/63(2006.01)i;  A61K 39/395(2006.01)i;  A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNTXT, WOTXT, USTXT, JPTXT, KRTXT, VEN, CNKI, 百度学术, pubmed, ISI web of science, STN, China Biological Sequence Search System: 信达生物制药（苏州）有限公司, 周帅祥, Claudin-18, Claudin 18, 同种型2, Claudin 18.2, CLDN18.2, HB37A6, CD3, 抗体, sp34, HzSP34.24, HzSP34.87, HzSP34.97, 人源化, 亲和力优化, 双特异性, bispecific, 序列1-27, SP34, affinity, Humanization, humanized, 紧密连接蛋白, 序列2/4/9/22/27/30/32

**C. DOCUMENTS CONSIDERED TO BE RELEVANT**

| Category* | Citation of document, with indication, where appropriate, of the relevant passages | Relevant to claim No. |
|---|---|---|
| X | CN 106589127 A (JUCHUAN BIOLOGY MEDICINE) 26 April 2017 (2017-04-26) description paragraphs 16, 22, 62-65, 69 and 72, tables 1-2 and SEQ ID NO.37 and SEQ ID NO.50 | 13-16 (in part) |
| X | CN 104968367 A (F. HOFFMANN-LA ROCHE LTD.) 07 October 2015 (2015-10-07) description sequence table SEQ ID NO.239 | 13-16( section ) |
| X | WO 2019213331 A1 (AMBRX, INC.) 07 November 2019 (2019-11-07) description, embodiment 8 | 13-16 (in part) |
| X | WO 2019075405 A1 (CYTOMX THERAPEUTICS INC.) 18 April 2019 (2019-04-18) description paragraphs 59-62 and table 1 | 13-16 (in part) |
| A | ZHU, G.Y. et al. "Targeting CLDN18.2 by CD3 Bispecific and ADC Modalities for the Treatments of Gastric and Pancreatic Cancer" *SCIENTIFIC REPORTS*, Vol. 1, No. 9, 10 June 2019 (2019-06-10), no. 8420 | 1-21 |
| A | CN 105073776 A (MICRO NUTRIENT LLC et al.) 18 November 2015 (2015-11-18) description, embodiment 1 | 1-21 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 December 2021** | **06 January 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/121286** |

**C.      DOCUMENTS CONSIDERED TO BE RELEVANT**

| Category* | Citation of document, with indication, where appropriate, of the relevant passages | Relevant to claim No. |
|---|---|---|
| A | ROOT A.R. et al. "Development of PF-06671008, a Highly Potent Anti-P-cadherin/Anti-CD3 Bispecific DART Molecule with Extended Half-Life for the Treatment of Cancer" *ANTIBODIES (BASEL)*, Vol. 5, No. 1, 04 March 2016 (2016-03-04), ISSN: 1,<br>       no. antib5010006 | 1-21 |
| A | WO 2018219901 A1 (F. HOFFMANN-LA ROCHE AG) 06 December 2018 (2018-12-06) description page 21 paragraph 4 and page 22 paragraph 4 | 1-21 |

Form PCT/ISA/210 (second sheet) (January 2015)

**INTERNATIONAL SEARCH REPORT**

| International application No. |
| --- |
| **PCT/CN2021/121286** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2021/121286**

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **19-21**
   because they relate to subject matter not required to be searched by this Authority, namely:

   [1]  Claims 19-21 relate to a method for preventing or treating tumors in a patient, which belongs to methods for treating diseases under PCT Rule 39.1(iv). Therefore, a search has been performed on the basis of a corresponding pharmaceutical use of the products in claims 19-21.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

PCT/CN2021/121286

| Patent document cited in search report | | | Publication date (day/month/year) | Patent family member(s) | | | Publication date (day/month/year) |
|---|---|---|---|---|---|---|---|
| CN | 106589127 | A | 26 April 2017 | CN | 108350085 | A | 31 July 2018 |
| | | | | US | 10858446 | B2 | 08 December 2020 |
| | | | | JP | 2018537958 | A | 27 December 2018 |
| | | | | EP | 3362482 | A1 | 22 August 2018 |
| | | | | JP | 6700387 | B2 | 27 May 2020 |
| | | | | US | 2018355059 | A1 | 13 December 2018 |
| | | | | HK | 1255331 | A1 | 16 August 2019 |
| | | | | EP | 3362482 | A4 | 07 August 2019 |
| | | | | WO | 2017063593 | A1 | 20 April 2017 |
| | | | | CN | 108350085 | B | 22 June 2021 |
| CN | 104968367 | A | 07 October 2015 | CL | 2015001266 | A1 | 28 August 2015 |
| | | | | EA | 201590731 | A1 | 30 November 2015 |
| | | | | ES | 2701746 | T3 | 25 February 2019 |
| | | | | US | 9284365 | B2 | 15 March 2016 |
| | | | | JP | 6538216 | B2 | 03 July 2019 |
| | | | | AR | 093446 | A1 | 10 June 2015 |
| | | | | WO | 2014078268 | A8 | 22 January 2015 |
| | | | | TW | I613214 | B | 01 February 2018 |
| | | | | IL | 238684 | A | 30 June 2015 |
| | | | | BR | 112015010817 | A2 | 28 November 2017 |
| | | | | TW | 201829457 | A | 16 August 2018 |
| | | | | JP | 6386465 | B2 | 05 September 2018 |
| | | | | EP | 2919813 | B1 | 24 October 2018 |
| | | | | SG | 10201700488 Q | A | 27 February 2017 |
| | | | | JP | 2018108086 | A | 12 July 2018 |
| | | | | TW | I657095 | B | 21 April 2019 |
| | | | | EA | 201892509 | A1 | 30 April 2019 |
| | | | | EP | 3461501 | A1 | 03 April 2019 |
| | | | | PE | 20150945 | A1 | 26 June 2015 |
| | | | | IL | 263320 | D0 | 31 December 2018 |
| | | | | MA | 38176 | A1 | 30 June 2017 |
| | | | | US | 2019023770 | A1 | 24 January 2019 |
| | | | | JP | 2016503413 | A | 04 February 2016 |
| | | | | WO | 2014078268 | A3 | 27 November 2014 |
| | | | | KR | 20150083124 | A | 16 July 2015 |
| | | | | AU | 2013345072 | B2 | 07 December 2017 |
| | | | | CN | 104968367 | B | 13 April 2018 |
| | | | | WO | 2014078268 | A2 | 22 May 2014 |
| | | | | HK | 1215856 | A1 | 23 September 2016 |
| | | | | MX | 2015005860 | A | 10 August 2015 |
| | | | | BR | 112015010817 | A8 | 23 January 2018 |
| | | | | US | 9598481 | B2 | 21 March 2017 |
| | | | | US | 9908931 | B2 | 06 March 2018 |
| | | | | US | 2016168230 | A1 | 16 June 2016 |
| | | | | SG | CN 11201503734 U | A | 29 June 2015 |
| | | | | US | 2014248286 | A1 | 04 September 2014 |
| | | | | EP | 2919813 | A2 | 23 September 2015 |
| | | | | UA | 116999 | C2 | 11 June 2018 |
| | | | | TW | 201420599 | A | 01 June 2014 |

Form PCT/ISA/210 (patent family annex) (January 2015)

**INTERNATIONAL SEARCH REPORT**
Information on patent family members

International application No.

**PCT/CN2021/121286**

| Patent document cited in search report | | | Publication date (day/month/year) | Patent family member(s) | | | Publication date (day/month/year) |
|---|---|---|---|---|---|---|---|
| | | | | CA | 2890669 | A1 | 22 May 2014 |
| | | | | IL | 238684 | D0 | 30 June 2015 |
| | | | | US | 2015259400 | A1 | 17 September 2015 |
| | | | | US | 2014161822 | A1 | 12 June 2014 |
| | | | | ZA | 201503403 | B | 30 November 2016 |
| | | | | PH | 12015501059 | A1 | 27 July 2015 |
| | | | | CR | 20150247 | A | 11 June 2015 |
| | | | | US | 9067979 | B2 | 30 June 2015 |
| | | | | AU | 2013345072 | A1 | 04 June 2015 |
| WO | 2019213331 | A1 | 07 November 2019 | BR | 112020022288 | A2 | 23 February 2021 |
| | | | | SG | 11202010439V | A | 27 November 2020 |
| | | | | AU | 2019263303 | A1 | 24 December 2020 |
| | | | | US | 2021108213 | A1 | 15 April 2021 |
| | | | | CA | 3098910 | A1 | 07 November 2019 |
| | | | | KR | 20210005172 | A | 13 January 2021 |
| | | | | CN | 112313336 | A | 02 February 2021 |
| | | | | EP | 3788149 | A1 | 10 March 2021 |
| WO | 2019075405 | A1 | 18 April 2019 | CN | 111247171 | A | 05 June 2020 |
| | | | | US | 2019135943 | A1 | 09 May 2019 |
| | | | | IL | 273937 | D0 | 31 May 2020 |
| | | | | CA | 3078911 | A1 | 18 April 2019 |
| | | | | SG | 11202002384V | A | 29 April 2020 |
| | | | | AU | 2018347607 | A1 | 26 March 2020 |
| | | | | EP | 3694885 | A1 | 19 August 2020 |
| | | | | KR | 20200064096 | A | 05 June 2020 |
| | | | | TW | 201927816 | A | 16 July 2019 |
| | | | | BR | 112020007309 | A2 | 29 September 2020 |
| | | | | JP | 2020536549 | A | 17 December 2020 |
| CN | 105073776 | A | 18 November 2015 | CN | 110144012 | A | 20 August 2019 |
| | | | | US | 2016272711 | A1 | 22 September 2016 |
| | | | | RU | 2015122484 | A | 10 January 2017 |
| | | | | NZ | 707831 | A | 30 November 2018 |
| | | | | JP | 2019162104 | A | 26 September 2019 |
| | | | | IL | 238464 | D0 | 30 June 2015 |
| | | | | AU | 2018211278 | B2 | 28 March 2019 |
| | | | | KR | 20150125642 | A | 09 November 2015 |
| | | | | JP | 6499079 | B2 | 10 April 2019 |
| | | | | ZA | 201502737 | B | 27 September 2017 |
| | | | | RU | 2019100107 | A | 25 February 2019 |
| | | | | JP | 2016500059 | A | 07 January 2016 |
| | | | | CN | 105073776 | B | 12 April 2019 |
| | | | | MX | 2015006003 | A | 09 December 2015 |
| | | | | US | 2019055311 | A1 | 21 February 2019 |
| | | | | KR | 20210043008 | A | 20 April 2021 |
| | | | | HK | 1209434 | A1 | 01 April 2016 |
| | | | | IL | 238464 | A | 31 May 2021 |
| | | | | AU | 2013347184 | A8 | 28 May 2015 |
| | | | | SG | CN 11201503593 U | A | 29 June 2015 |
| | | | | JP | 6799101 | B2 | 09 December 2020 |

Form PCT/ISA/210 (patent family annex) (January 2015)

## INTERNATIONAL SEARCH REPORT
### Information on patent family members

International application No.

**PCT/CN2021/121286**

| Patent document cited in search report | | | Publication date (day/month/year) | Patent family member(s) | | | Publication date (day/month/year) |
|---|---|---|---|---|---|---|---|
| | | | | AU | 2013347184 | B2 | 14 June 2018 |
| | | | | JP | 2021040641 | A | 18 March 2021 |
| | | | | MX | 369276 | B | 04 November 2019 |
| | | | | UA | 117741 | C2 | 25 September 2018 |
| | | | | NZ | 746691 | A | 28 August 2020 |
| | | | | RU | 2678127 | C2 | 23 January 2019 |
| | | | | AU | 2018211278 | A1 | 23 August 2018 |
| | | | | WO | 2014075788 | A1 | 22 May 2014 |
| | | | | CA | 2890438 | A1 | 22 May 2014 |
| | | | | AU | 2013347184 | A1 | 21 May 2015 |
| | | | | KR | 102240664 | B1 | 15 April 2021 |
| | | | | MX | 2019012846 | A | 28 November 2019 |
| | | | | US | 10093736 | B2 | 09 October 2018 |
| | | | | US | 10717780 | B2 | 21 July 2020 |
| | | | | US | 2020399370 | A1 | 24 December 2020 |
| WO | 2018219901 | A1 | 06 December 2018 | MX | 2019014291 | A | 27 January 2020 |
| | | | | EP | 3630290 | A1 | 08 April 2020 |
| | | | | IL | 270867 | D0 | 30 January 2020 |
| | | | | BR | 112019025062 | A2 | 30 June 2020 |
| | | | | AU | 2018276345 | A1 | 05 December 2019 |
| | | | | CN | 110678228 | A | 10 January 2020 |
| | | | | CA | 3064668 | A1 | 06 December 2018 |
| | | | | EP | 3409322 | A1 | 05 December 2018 |
| | | | | KR | 20200014345 | A | 10 February 2020 |
| | | | | US | 2020199230 | A1 | 25 June 2020 |
| | | | | TW | 201902513 | A | 16 January 2019 |
| | | | | JP | 2020521798 | A | 27 July 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010570517X

- US 8236308 B

### Non-patent literature cited in the description

- **SINGH, P. ; TOOM, S. ; HUANG, Y.** Anti-CLDN18.2 antibody as new targeted therapy for advanced gastric cancer. *J Hematol Oncol,* 2017, vol. 10, 105, https://doi.org/10.1186/s13045-017-0473-4
- **O. TURECI. et al.** *Gene,* 2011, vol. 481, 83-92
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987
- **KABAT et al.** Sequences of Proteins of Immunological Interest. Public Health Service, National Institutes of Health, 1991

- **NEEDLEMA ; WUNSCH.** *J. Mol. Biol.,* 1970, vol. 48, 444-453
- *CABIOS,* 1989, vol. 4, 11-17
- Current Protocols in Molecular Biology. John Wiley & Sons, 1989, 6.3.1-6.3.6
- **SHANE ATWELL et al.** *Journal of Molecular Biology,* 1997
- **A. MARGARET MERCHANT et al.** *Nature Biotechnology,* 1998
- **R.C. ROWE ; P.J. SESKEY ; S.C. OWEN.** Handbook of Pharmaceutical Excipients. Pharmaceutical Press
- *J. Immunol. Methods.,* 1994, vol. 178, 195
- **ESTEP, P et al.** High throughput solution based measurement of antibody-antigen affinity and affinity binding. *MAbs,* 2013, vol. 5 (2), 270-8